# EUROPEAN PATENT APPLICATION

(11) **EP 4 831 916 A2**
(43) Date of publication of application: **28.10.2026**
(21) Application number: 26192322.1
(22) Date of filing: 24.08.2023
(51) Int. Cl.: G06T 7/11

(54) **SEGMENTING AND DETECTING AMYLOID-RELATED IMAGING ABNORMALITES (ARIA) IN ALZHEIMER S PATIENTS**

(30) Priority: 25.08.2022 US 202263401038 P
(62) Document number(s) of the earlier application(s) in accordance with Art. 76 EPC: 23773104.7 / 4 577 977
(71) Applicant: GENENTECH, INC., South San Francisco, CA 94080-4990 (US); F. Hoffmann-La Roche AG, 4070 Basel (CH)
(72) Inventor: KLEIN, Gregory, 4070 Basel (CH); KRISHNAN, Anitha Priya, San Francisco, 94080-4990 (US); SONG, Zhuang, San Francisco, 94080-4990 (US); CARANO, Richard Alan Duray, San Francisco, 94080-4990 (US)
(74) Representative: Mewburn Ellis LLP

(57) **Abstract**

Methods for segmenting and detecting amyloid related imaging abnormalities (ARIA) in a brain of a patient are provided. The method includes accessing a set of one or more brain-scan images associated with the patient, and inputting the set of one or more brain-scan images into one or more machine-learning models trained to generate a segmentation map based on the set of one or more brain-scan images. The segmentation map includes a plurality of pixel-wise class labels corresponding to a plurality of pixels in the segmentation map, in which at least one of the plurality of pixel-wise class labels includes an indication of ARIA in the brain of the patient. The method further includes outputting a quantification of ARIA in the brain of the patient based at least in part on the segmentation map.

## Description

### CROSS-REFERENCE TO RELATED APPLICATIONS

This application claims priority to U.S. Provisional Application No. 63/401,038, entitled "Segmenting and Detecting Amyloid-Related Imaging Abnormalities (ARIA) in Alzheimer's Patients," which was filed on August 25, 2022, and the disclosure of which is hereby incorporated by reference in its entirety.

### TECHNICAL FIELD

The present disclosure relates generally to amyloid-related imaging abnormalities (ARIA), and, more specifically, to segmenting and detecting ARIA in Alzheimer's disease (AD) patients.

### BACKGROUND

Alzheimer's disease (AD) is a progressive neurodegenerative disease that may be characterized by a decline in patient memory, speech, and cognitive skills, as well as by adverse changes in patient mood and behavior. AD may generally result from one or more identified biological changes that may occur in the brain of the patient over many years. For example, leading biological markers (e.g., biomarkers) or hallmarks of AD may include the excessive accumulation of amyloid-beta (Aβ) plaques and tau tangles within the brain of the patient. Specifically, while Aβ proteins and tau proteins may be produced generally as part of the normative functioning of the brain, in patients diagnosed with AD, one may observe either an excessive production of Aβ proteins that may accumulate as plaques around the brain cells or an excessive production of tau proteins that may become misfolded and accumulate as tangles within the brain cells. For example, the Aβ plaques or tau tangles may be typically observed in a patient's brain by performing one or more magnetic resonance imaging (MRI) scans, positron-emission tomography (PET) scans, or computed tomography (CT) scans of the patient's brain, and then these scans may be utilized by clinicians to diagnose patients as having AD.

In certain instances, for patients diagnosed with AD, when excessive accumulation of Aβ plaques is the basis for the diagnosis (e.g., as opposed to the accumulation of tau tangles), clinicians may treat the AD patient utilizing an anti-amyloid-beta (anti-Aβ) antibody or other similar anti-Aβ immunotherapy. For example, the anti-Aβ antibody may include one or more anti-Aβ monoclonal antibodies (mAbs) that may be suitable for removing or reducing Aβ plaques in the brain of the AD patient by binding to and counteracting the Aβ plaques. While such anti-Aβ antibody treatments have been found to be effective for treating AD patients, in a small number of instances, an AD patient may be susceptible to certain side effects from the anti-Aβ antibody treatments that may manifest as amyloid-related imaging abnormalities (ARIA) in subsequent scans (e.g., MRI scans, PET scans) of the brain of the AD patient. For example, ARIA may include ARIA-E, which includes parenchymal or sulcal hyperintensities on certain MRI scans (e.g., fluid-attenuated inversion recovery (FLAIR) imaging) indicative of parenchymal edema or sulcal effusions. ARIA may further include ARIA-H, which includes hypointense regions on other particular MRI scans (e.g., gradient recalled-echo imaging, T2*-weighted imaging (T2*WI)) indicative of hemosiderin deposition. It may be thus useful to detect ARIA as early as possible, such that the anti-Aβ antibody treatments may be adjusted and/or temporarily suspended in such instances in which an AD patient shows signs of ARIA. Accordingly, it may be useful to provide techniques for analyzing brain scans to detect and quantify ARIA, which may manifest as contextual changes and/or changes in signal intensities in the brain scans.

### SUMMARY

Embodiments of the present disclosure are directed to one or more computing devices, methods, and non-transitory computer-readable media that may utilize one or more machine-learning models (e.g., one or more semantic image segmentation and classification models) for analyzing medical images (e.g., brain-scan images) to segment, detect, and quantify amyloid-related imaging abnormalities (ARIA) in Alzheimer's disease (AD) patients. For example, in certain embodiments, the one or more computing devices may access a set of one or more brain-scan images (e.g., magnetic resonance imaging (MRI) scans, positron-emission tomography (PET) scans) associated with an AD patient and input the set of one or more brain-scan images into one or more machine-learning models (e.g., one or more semantic image segmentation and classification models). The one or more machine-learning models (e.g., one or more semantic image segmentation and classification models) may be trained to generate a segmentation map based on the set of one or more brain-scan images and one or more classification scores based on the segmentation map. For example, in certain embodiments, the segmentation map may include a plurality of pixel-wise class labels or voxel-wise class labels corresponding to a plurality of pixels or voxels in the segmentation map, in which at least one of the plurality of pixel-wise class labels or voxel-wise class labels includes an indication (e.g., an area corresponding to one or more ARIA lesions) of ARIA in the brain of the patient. In certain embodiments, the one or more machine-learning models (e.g., one or more semantic image segmentation and classification models) may then generate one or more classification scores based on the segmentation map, in which the one or more classification scores may indicate a presence of ARIA and/or a severity of ARIA.

Specifically, in accordance with the presently disclosed embodiments, the one or more machine-learning models (e.g., one or more semantic image segmentation and classification models) may segment pixels or voxels of the input brain scans on a pixel-by-pixel or voxel-by-voxel basis and generate a segmentation map in which the pixels or voxels corresponding to areas of the patient's brain (e.g., deposition of Aβ proteins in the folds of the brain and/or diffuse swelling) are classified as being indicative of ARIA and/or generate one or more classification scores for the patient at a given time point indicating a detection (e.g., presence of ARIA or absence of ARIA) or severity of ARIA (e.g., mild ARIA, moderate ARIA, severe ARIA) based on the segmentation map. For example, in some embodiments, the one or more machine-learning models may include only a segmentation model trained to generate a prediction of a segmentation map, which may include a pixel-wise or voxel-wise semantic segmentation of one or more ARIA lesions (e.g., deposition of Aβ proteins in the folds of the brain and/or diffuse swelling) apparent in the brain scans of the brain of the patient.

In other embodiments, the one or more machine-learning models may include a joint segmentation model and classification model trained in accordance with a multi-task learning process, in which a classification arm may be added to the segmentation model. The multi-task learning process may be provided to improve machine-learning model performance by learning shared representations and reducing the possibility of overfitting the machine-learning model. Here, the classification and segmentation tasks share the features extracted by the encoder of the machine-learning model, enabling robust selection of features across tasks and improving segmentation performance. However, the joint segmentation model and classification model may also include more parameters than the segmentation model alone. This may lead to challenges with respect accurately training the joint segmentation model and classification model utilizing only a limited training dataset.

Accordingly, in certain embodiments, two separate models may be trained separately for the segmentation task and the classification task, respectively. For example, in certain embodiments, the separate segmentation model and the classification model may be trained in accordance with a transfer learning process, in which a set of weights learned by way of the training of the encoder of the segmentation model may be utilized to initialize the set of weights of the classification model. In certain embodiments, the classification model may be further pre-trained in accordance with one or more contrastive learning processes (e.g., supervised contrastive learning, self-supervised contrastive learning), in which the classification model may be in part pre-trained to generate a classification score based on the set of one or more brain-scan images, indicating the presence or absence of ARIA in the patient's brain at a given time point. After the pre-training of the classification model, the last few layers of the classification model may be further trained and/or fine-tuned for the classification score that may indicate the severity of ARIA (e.g., mild ARIA, moderate ARIA, severe ARIA) in the brain of the patient more generally.

Indeed, the present embodiments may provide techniques to accurately segment and classify brain scans (e.g., MRI scans, PET scans) for segmenting, detecting, and quantifying ARIA, which may manifest as contextual changes and/or changes in signal intensities in the brain scans (e.g., MRI scans, PET scans). The present embodiments may further provide techniques to train the one or more machine-learning models (e.g., one or more semantic image segmentation and classification models) to accurately segment and classify brain scans for segmenting, detecting, and quantifying ARIA utilizing only a limited training dataset (e.g., as ARIA may be observed clinically in only a small subgroup of AD patients of a much larger group of AD patients having been treated utilizing anti-Aβ monoclonal antibodies (mAbs)).

Specifically, pixel-wise or voxel-wise annotation of ARIA lesions by way of human annotators may be time-consuming, costly, and immensely susceptible to error. Hence, such annotations are usually acquired on only a limited dataset, while less complex annotations for ARIA scores per visit/time point at the patient level may be relatively easier to acquire on a larger dataset. To account for the differences in the availability of pixel-wise or voxel-wise and scan/visit level annotations, the present embodiments may provide techniques to not only train and utilize a joint segmentation and classification model to accurately segment and classify brain scans (e.g., MRI scans, PET scans) for detecting and quantifying ARIA, but, alternatively, to train and utilize distinct models to 1) segment the brain scans (e.g., MRI scans, PET scans) to identify ARIA lesions, and another distinct classification model to 2) classify the brain scans (e.g., MRI scans, PET scans) by predicting ARIA scores corresponding to a presence or severity of the identified ARIA lesions. In this way, when sufficient training data (e.g., ground truth data of both pixel-wise or voxel-wise annotated images and ARIA scoring) for accurately training the joint segmentation and classification model is not readily available, the present embodiments may provide techniques to separately train and utilize a distinct segmentation model and a distinct classification model for segmenting, detecting, and quantifying ARIA.

The present embodiments described herein may further provide a number of technical advantages. For example, the implementation of the one or more machine-learning models may be memory-efficient in that an entire set of 3-dimensional (3D) images corresponding to one or more volumetric structures (e.g., a set of voxels representing slices of the patient's brain) may be the input to the one or more machine-learning models. This may allow the one or more machine-learning models to be easily fine-tuned for downstream tasks. Further, the one or more machine-learning models may enable easy flow of information from local size scale to global size scale and incorporate both global and local information. This thus provides more accurate segmentation results because ARIA information may be generally local and relatively small in size (e.g., in terms of area). Further, the one or more machine-learning models may include a relatively more intensive encoder and a relatively less intensive decoder, such that decoding may be performed efficiently. For at least these foregoing reasons, the design and implementation of the one or more machine-learning models described herein may improve the functioning of a computer by requiring less memory, processing power, and power consumption.

In certain embodiments, in response to detecting ARIA in the brain of the patient, the one or more computing devices may determine a dosage adjustment of the anti-Aβ antibody treatment. In some embodiments, if ARIA is detected, the one or more computing devices may recommend a reduced dosage of the anti-Aβ antibody. The recommendation may be provided via one or more outputs (e.g., visual, auditory, haptic outputs), by generating a report for a clinician, etc.

In some embodiments, if ARIA is detected, the one or more computing devices may determine a reduced dosage of the anti-Aβ antibody. For example, the one or more computing devices may compare the results of the one or more machine-learning models to one or more predefined thresholds to determine the severity of ARIA. In accordance with a determination that the detected ARIA is mild, the one or more computing devices may determine a first reduced dosage. In accordance with a determination that the detected ARIA is severe, the one or more computing devices may determine a second reduced dosage lower than the first reduced dosage. The determined dosage may be provided via one or more outputs (e.g., visual, auditory, haptic outputs), by generating a report for a clinician, etc. In some embodiments, the one or more computing devices may automatically control a medical device to administer the reduced dosage of the anti-Aβ antibody to the patient.

In some embodiments, if ARIA is detected, the one or more computing devices may determine to terminate or temporarily suspend the prescription or administration of the anti-Aβ antibody to the patient. For example, the one or more computing devices may compare results of the one or more machine-learning models to one or more predefined criteria to determine if the anti-Aβ antibody should be terminated or temporarily suspended. The termination or suspension decision may be provided via one or more outputs (e.g., visual, auditory, haptic outputs), by generating a report for a clinician, etc. In some embodiments, the one or more computing devices may automatically control a medical device to terminate or temporarily suspend the administration of the anti-Aβ antibody to the patient.

In some embodiments, if ARIA is detected, the one or more computing devices may determine one or more anti-ARIA treatments (e.g., one or more anti-ARIA antibodies) for the patient. For example, the one or more computing devices may compare results of the one or more machine-learning models to one or more predefined thresholds to determine the recommended treatment. The identified treatments may be provided via one or more outputs (e.g., visual, auditory, haptic outputs), by generating a report for a clinician, etc. In some embodiments, the one or more computing devices may automatically control a medical device to administer the anti-ARIA treatments to the patient.

The one or more computing devices may monitor ARIA in a patient over time. In some embodiments, the one or more computing devices may be configured to receive different sets of medical images corresponding to different time points and analyze the images using the techniques described herein. By monitoring ARIA in the patient over time, the one or more computing devices may determine whether any of the responses above (e.g., reduced dosage, terminated or temporarily suspended administration, anti-ARIA treatments) is effective, and formulate an adjusted response accordingly. In some embodiments, the one or more computing devices may monitor ARIA in multiple patients that have received different types of anti-Aβ antibodies and, by comparing the presence and/or severity of ARIA in these patients over time, determine the safeness of these different types of anti-Aβ antibodies to inform future treatment decisions.

### BRIEF DESCRIPTION OF THE DRAWINGS

FIG. 1A illustrates an MRI scan of a patient's brain before the patient was treated with an anti-Aβ antibody, in accordance with some embodiments.
FIG. 1B illustrates an MRI scan of a patient's brain after the patient was treated with an anti-Aβ antibody, in accordance with some embodiments.
FIG. 2A illustrates an MRI scan of a patient's brain before the patient was treated with an anti-Aβ antibody, in accordance with some embodiments.
FIG. 2B illustrates an MRI scan of a patient's brain after the patient was treated with an anti-Aβ antibody, in accordance with some embodiments.
FIG. 3A illustrates an exemplary process for segmenting ARIA in a patient, in accordance with some embodiments.
FIG. 3B illustrates an exemplary process for detecting ARIA in a patient, in accordance with some embodiments.
FIG. 4 illustrates an exemplary trained segmentation model, in accordance with some embodiments.
FIG. 5 illustrates an exemplary trained joint segmentation/classification model, in accordance with some embodiments.
FIG. 6 illustrates another exemplary trained joint segmentation/classification model, in accordance with some embodiments.
FIG. 7 illustrates an exemplary process for separately training a segmentation model and a classification model, in accordance with some embodiments.
FIG. 8A illustrates an exemplary trained classification model, in accordance with some embodiments.
FIG. 8B illustrates an exemplary pre-trained classification model, in accordance with some embodiments.
FIG. 9 illustrates an exemplary process for pre-training a classification model, in accordance with some embodiments.
FIG. 10 illustrates exemplary training images used for contrastive learning, in accordance with some embodiments.
FIG. 11 illustrates an exemplary stack of 32 MRI slices of a patient's brain in 3D with slices corresponding to different cross sections of the brain, in accordance with some embodiments.
FIG. 12 illustrates an exemplary stack of 32 MRI slices of a patient's brain in 3D with slices corresponding to different cross sections of the brain, in accordance with some embodiments.
FIG. 13 illustrates an example computing system.
FIG. 14 illustrates a diagram of an example artificial intelligence (AI) architecture included as part of the example computing system of FIG. 13.

### DESCRIPTION OF EXAMPLE EMBODIMENTS

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

Embodiments of the present disclosure are directed to one or more computing devices, methods, and non-transitory computer-readable media that may utilize one or more machine-learning models (e.g., one or more semantic image segmentation and classification models) for analyzing medical images (e.g., brain-scan images) to segment, detect, and quantify amyloid-related imaging abnormalities (ARIA) in Alzheimer's disease (AD) patients. For example, in certain embodiments, the one or more computing devices may access a set of one or more brain-scan images (e.g., magnetic resonance imaging (MRI) scans, positron-emission tomography (PET) scans) associated with an AD patient and input the set of one or more brain-scan images into one or more machine-learning models (e.g., one or more semantic image segmentation and classification models). The one or more machine-learning models (e.g., one or more semantic image segmentation and classification models) may be trained to generate a segmentation map based on the set of one or more brain-scan images and one or more classification scores based on the segmentation map. For example, in certain embodiments, the segmentation map may include a plurality of pixel-wise class labels or voxel-wise class labels corresponding to a plurality of pixels or voxels in the segmentation map, in which at least one of the plurality of pixel-wise class labels or voxel-wise class labels includes an indication (e.g., an area corresponding to one or more ARIA lesions) of ARIA in the brain of the patient. In certain embodiments, the one or more machine-learning models (e.g., one or more semantic image segmentation and classification models) may then generate one or more classification scores based on the segmentation map, in which the one or more classification scores may indicate a presence of ARIA and/or a severity of ARIA.

Specifically, in accordance with the presently disclosed embodiments, the one or more machine-learning models (e.g., one or more semantic image segmentation and classification models) may segment pixels or voxels of the input brain scans on a pixel-by-pixel or voxel-by-voxel basis and generate a segmentation map in which the pixels or voxels corresponding to areas of the patient's brain (e.g., deposition of Aβ proteins in the folds of the brain and/or diffuse swelling) are classified as being indicative of ARIA and/or generate one or more classification scores for the patient at a given time point indicating a detection (e.g., presence of ARIA or absence of ARIA) or severity of ARIA (e.g., mild ARIA, moderate ARIA, severe ARIA) based on the segmentation map. For example, in some embodiments, the one or more machine-learning models may include only a segmentation model trained to generate a prediction of a segmentation map, which may include a pixel-wise or voxel-wise semantic segmentation of one or more ARIA lesions (e.g., deposition of Aβ proteins in the folds of the brain and/or diffuse swelling) apparent in the brain scans of the brain of the patient.

In other embodiments, the one or more machine-learning models may include a joint segmentation model and classification model trained in accordance with a multi-task learning process, in which a classification arm may be added to the segmentation model. The multi-task learning process may be provided to improve machine-learning model performance by learning shared representations and reducing the possibility of overfitting the machine-learning model. Here, the classification and segmentation tasks share the features extracted by the encoder of the machine-learning model, enabling robust selection of features across tasks and improving segmentation performance. However, the joint segmentation model and classification model may also include more parameters than the segmentation model alone. This may lead to challenges with respect accurately training the joint segmentation model and classification model utilizing only a limited training dataset.

Accordingly, in certain embodiments, two separate models may be trained separately for the segmentation task and the classification task, respectively. For example, in certain embodiments, the separate segmentation model and the classification model may be trained in accordance with a transfer learning process, in which a set of weights learned by way of the training of the encoder of the segmentation model may be utilized to initialize the set of weights of the classification model. In certain embodiments, the classification model may be further pre-trained in accordance with one or more contrastive learning processes (e.g., supervised contrastive learning, self-supervised contrastive learning), in which the classification model may be in part pre-trained to generate a classification score based on the set of one or more brain-scan images, indicating the presence or absence of ARIA in the patient's brain at a given time point. After the pre-training of the classification model, the last few layers of the classification model may be further trained and/or fine-tuned for the classification score that may indicate the severity of ARIA (e.g., mild ARIA, moderate ARIA, severe ARIA) in the brain of the patient more generally.

Indeed, the present embodiments may provide techniques to accurately segment and classify brain scans (e.g., MRI scans, PET scans) for segmenting, detecting, and quantifying ARIA, which may manifest as contextual changes and/or changes in signal intensities in the brain scans (e.g., MRI scans, PET scans). The present embodiments may further provide techniques to train the one or more machine-learning models (e.g., one or more semantic image segmentation and classification models) to accurately segment and classify brain scans for segmenting, detecting, and quantifying ARIA utilizing only a limited training dataset (e.g., as ARIA may be observed clinically in only a small subgroup of AD patients of a much larger group of AD patients having been treated utilizing anti-Aβ monoclonal antibodies (mAbs)).

Specifically, pixel-wise or voxel-wise annotation of ARIA lesions by way of human annotators may be time-consuming, costly, and immensely susceptible to error. Hence, such annotations are usually acquired on only a limited dataset, while less complex annotations for ARIA scores per visit/time point at the patient level may be relatively easier to acquire on a larger dataset. To account for the differences in the availability of pixel-wise or voxel-wise and scan/visit level annotations, the present embodiments may provide techniques to not only train and utilize a joint segmentation and classification model to accurately segment and classify brain scans (e.g., MRI scans, PET scans) for detecting and quantifying ARIA, but, alternatively, to train and utilize distinct models to 1) segment the brain scans (e.g., MRI scans, PET scans) to identify ARIA lesions, and another distinct classification model to 2) classify the brain scans (e.g., MRI scans, PET scans) by predicting ARIA scores corresponding to a presence or severity of the identified ARIA lesions. In this way, when sufficient training data (e.g., ground truth data of both pixel-wise or voxel-wise annotated images and ARIA scoring) for accurately training the joint segmentation and classification model is not readily available, the present embodiments may provide techniques to separately train and utilize a distinct segmentation model and a distinct classification model for segmenting, detecting, and quantifying ARIA.

The present embodiments described herein may further provide a number of technical advantages. For example, the implementation of the one or more machine-learning models may be memory-efficient in that an entire set of 3-dimensional (3D) images corresponding to one or more volumetric structures (e.g., a set of voxels representing slices of the patient's brain) may be the input to the one or more machine-learning models. This may allow the one or more machine-learning models to be easily fine-tuned for downstream tasks. Further, the one or more machine-learning models may enable easy flow of information from local size scale to global size scale and incorporate both global and local information. This thus provides more accurate segmentation results because ARIA information may be generally local and relatively small in size (e.g., in terms of area). Further, the one or more machine-learning models may include a relatively more intensive encoder and a relatively less intensive decoder, such that decoding may be performed efficiently. For at least these foregoing reasons, the design and implementation of the one or more machine-learning models described herein may improve the functioning of a computer by requiring less memory, processing power, and power consumption.

In certain embodiments, in response to detecting ARIA in the brain of the patient, the one or more computing devices may determine a dosage adjustment of the anti-Aβ antibody treatment (e.g., amyloid blocker drugs). In some embodiments, if ARIA is detected, the one or more computing devices may recommend a reduced dosage of the anti-Aβ antibody. The recommendation may be provided via one or more outputs (e.g., visual, auditory, haptic outputs), by generating a report for a clinician, etc.

In some embodiments, if ARIA is detected, the one or more computing devices may determine a reduced dosage of the anti-Aβ antibody. For example, the one or more computing devices may compare the results of the one or more machine-learning models to one or more predefined thresholds to determine the severity of ARIA. In accordance with a determination that the detected ARIA is mild, the one or more computing devices may determine a first reduced dosage. In accordance with a determination that the detected ARIA is severe, the one or more computing devices may determine a second reduced dosage lower than the first reduced dosage. The determined dosage may be provided via one or more outputs (e.g., visual, auditory, haptic outputs), by generating a report for a clinician, etc. In some embodiments, the one or more computing devices may automatically control a medical device to administer the reduced dosage of the anti-Aβ antibody to the patient.

In some embodiments, if ARIA is detected, the one or more computing devices may determine to terminate or temporarily suspend the prescription or administration of the anti-Aβ antibody to the patient. For example, the one or more computing devices may compare results of the one or more machine-learning models to one or more predefined criteria to determine if the anti-Aβ antibody should be terminated or temporarily suspended. The termination or suspension decision may be provided via one or more outputs (e.g., visual, auditory, haptic outputs), by generating a report for a clinician, etc. In some embodiments, the one or more computing devices may automatically control a medical device to terminate or temporarily suspend the administration of the anti-Aβ antibody to the patient.

In some embodiments, if ARIA is detected, the one or more computing devices may determine one or more anti-ARIA treatments (e.g., one or more anti-ARIA antibodies) for the patient. For example, the one or more computing devices may compare results of the one or more machine-learning models to one or more predefined thresholds to determine the recommended treatment. The identified treatments may be provided via one or more outputs (e.g., visual, auditory, haptic outputs), by generating a report for a clinician, etc. In some embodiments, the one or more computing devices may automatically control a medical device to administer the anti-ARIA treatments to the patient.

The one or more computing devices may monitor ARIA in a patient over time. In some embodiments, the one or more computing devices may be configured to receive different sets of medical images corresponding to different time points and analyze the images using the techniques described herein. By monitoring ARIA in the patient over time, the one or more computing devices may determine whether any of the responses above (e.g., reduced dosage, terminated or temporarily suspended administration, anti-ARIA treatments) is effective, and formulate an adjusted response accordingly. In some embodiments, the one or more computing devices may monitor ARIA in multiple patients that have received different types of anti-Aβ antibodies and, by comparing the presence and/or severity of ARIA in these patients over time, determine the safeness of these different types of anti-Aβ antibodies to inform future treatment decisions.

As used herein, a "pixel" may refer to the smallest unit of a two-dimensional (2D) digital image (e.g., 2D medical image), which may be illuminated on a display, such that a set of such illuminated "pixels" forms the complete 2D digital image (e.g., 2D medical image), for example. For example, in some instances, each "pixel" may include a unique geometric coordinate, XY dimensions, a size (e.g., which may be expressed in bits), and may be utilized to display one or more of a number of color values representative of the 2D digital image. Similarly, as used herein, a "voxel" may refer to the smallest distinguishable element of any three-dimensional (3D) volume (e.g., a 3D volume, such as a patient's brain or other human organ), and may be represented as a grid value in 3D space, for example. For example, in some instances, a "voxel" may be understood to be a "volume pixel" having XYZ dimensions, and thus a "pixel," as used herein, may be understood to encompass both 2D pixels and 3D voxels.

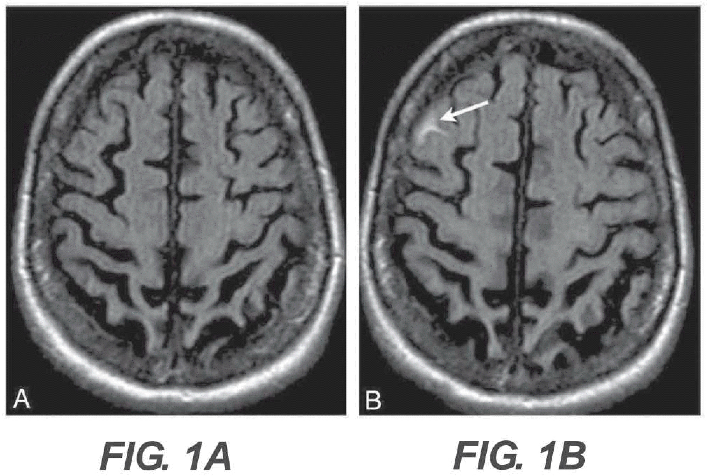

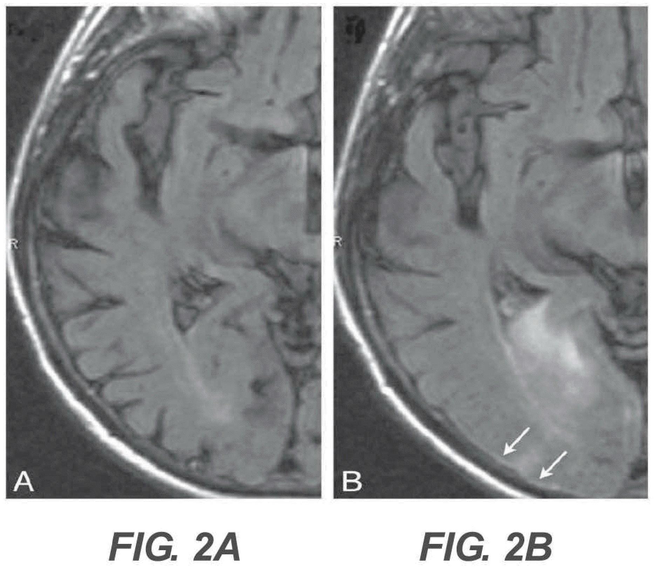

**FIGs. 1A-1B** and **2A-2B** illustrate that imaging features of ARIA may be challenging to segment, detect, and quantify in brain-scan images, such as magnetic resonance imaging (MRI) scans, positron-emission tomography (PET) scans, computed tomography (CT) scans, and so forth. For example, FIGs. 1A-1B may include MRI scans of an AD patient's brain before and after the AD patient had undergone treatment for AD utilizing an anti-amyloid-beta (anti-Aβ) antibody as discussed herein, respectively. The AD patient has suffered from brain lesions after taking the anti-Aβ antibody. As shown in FIG. 1B, the lesion effect is shown as a hypointense signal that occurs on the surface of the brain, indicated by the arrow. Due to the change in signal intensities, the lesion effect may be relatively easy to identify in the brain scan images by a visual review.

However, in contrast to a lesion effect, a swelling effect of the AD patient's brain may be comparatively challenging to segment, detect, and quantify. For example, FIGs. 2A-2B may include MRI scans of an AD patient's brain before and after the AD patient had undergone treatment for AD utilizing an anti-Aβ antibody as discussed herein, respectively. The AD patient has suffered from brain swellings after taking the anti-Aβ antibody. As shown in FIG. 2B, some folds in the brain of the AD patient that were present in FIG. 2A have disappeared due to, for example, the deposition of amyloid proteins in the folds of the brain and diffuse swelling in the brain. The areas of change are noted by the arrows in FIG. 2B. However, FIG. 2B does not depict any prominent changes in signal intensities, making the swelling effect comparatively challenging to segment, detect, and quantify even for trained clinicians (e.g., neurologists, radiologists, neurosurgeons) and thus leading to potential misdiagnosis and/or reduced inter-rater agreement.

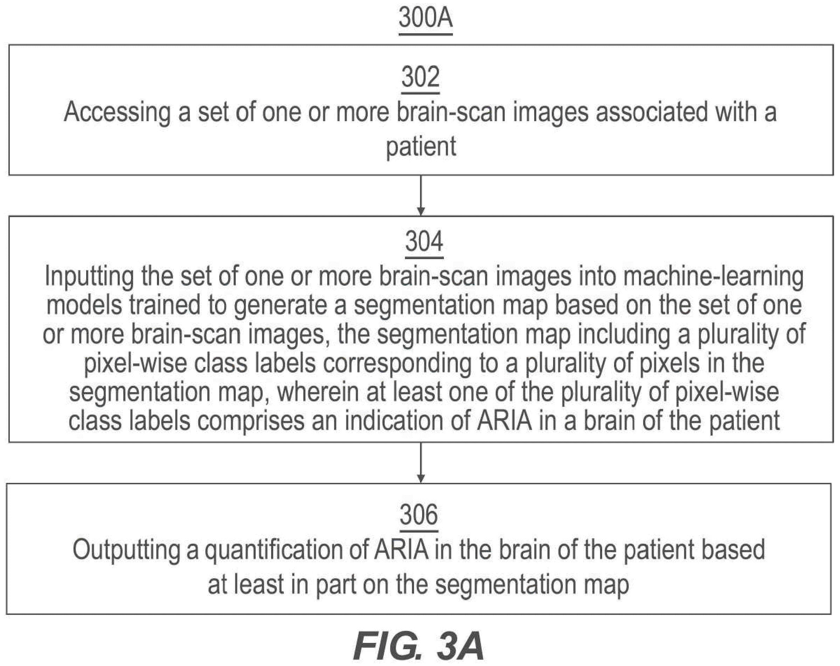

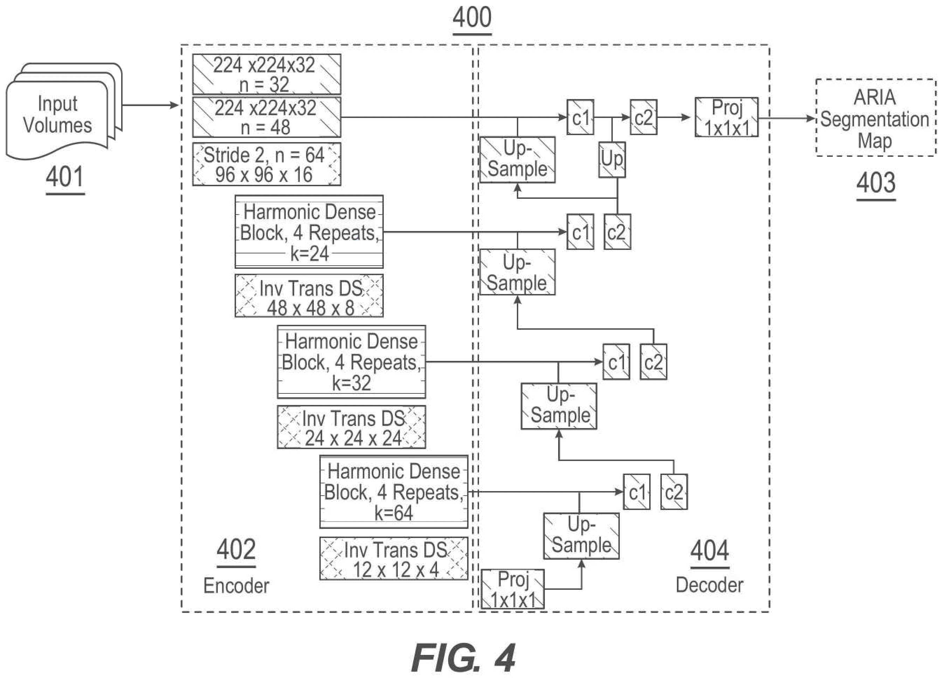

**FIG. 3A** illustrates an exemplary process 300A for segmenting and quantifying ARIA in the brain of a patient, according to various examples. In certain embodiments, the process 300A may be performed, for example, in accordance with the illustrated segmentation model 400 to be discussed below with respect to FIG. 4. Process 300A is performed, for example, using one or more electronic devices implementing a software platform. In some examples, process 300A is performed using a client-server system, and the blocks of process 300A are divided up in any manner between the server and one or more client devices. In other examples, process 300A is performed using only one or more client devices. In process 300A, some blocks are, optionally, combined, the order of some blocks is, optionally, changed, and some blocks are, optionally, omitted. In some examples, additional steps may be performed in combination with the process 300A. Accordingly, the operations as illustrated (and described in greater detail below) are exemplary by nature and, as such, should not be viewed as limiting.

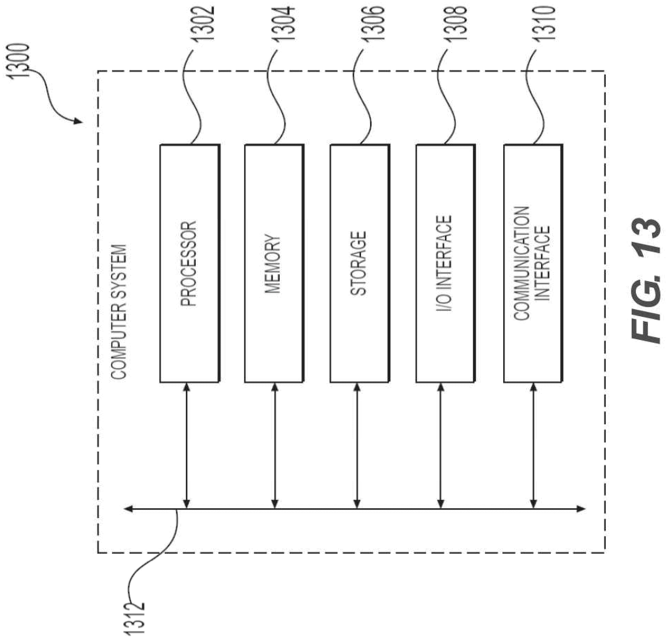

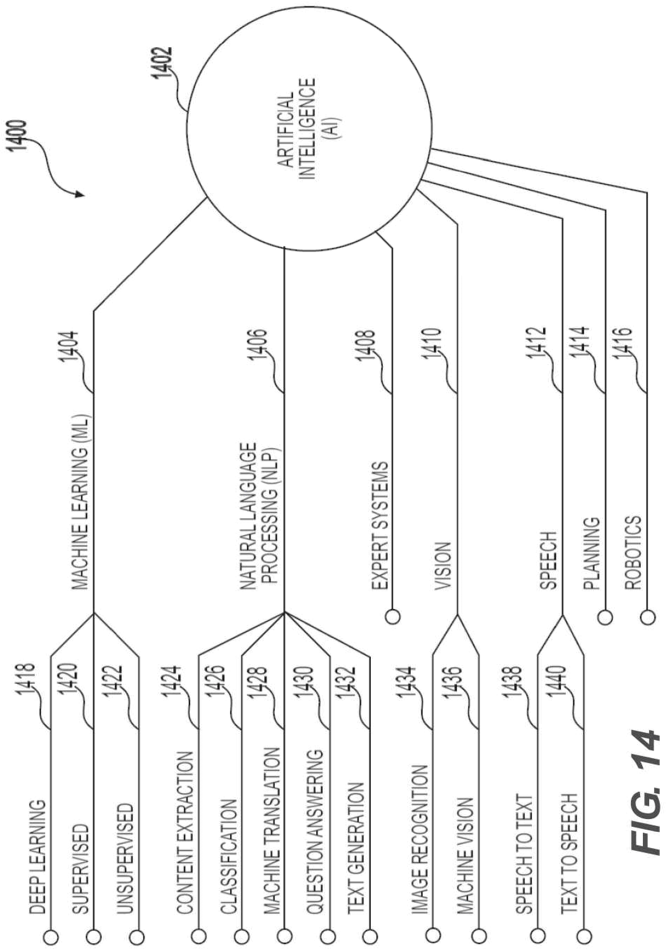

The process 300A may be performed utilizing one or more processing devices (e.g., computing system and artificial intelligence architecture to be discussed below with respect to FIGS. 13 and 14) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing various medical data and making one or more decisions based thereon), software (e.g., instructions running/executing on one or more processors), firmware (e.g., microcode), or some combination thereof.

At block 302, an exemplary system including one or more computing devices may access a set of one or more brain-scan images associated with the patient. The one or more computing devices may retrieve the one or more brain-scan images from one or more computer memories, from one or more imaging devices, from one or more local or remote databases, or any other data sources. The one or more computing devices may access the set images automatically or in response to a user input.

The set of one or more brain-scan images may be taken before, during, or after a treatment is administered to the patient. In some embodiments, the patient is an AD patient having been treated with an anti-Aβ antibody. For example, in certain embodiments, the anti-Aβ antibody may include one or more anti-Aβ monoclonal antibodies (mAbs) and/or one or more other similar anti-Aβ immunotherapies that may be suitable for removing or reducing Aβ plaques that may accumulate in the brain of an AD patient by binding to and counteracting the Aβ plaques. In one embodiment, the anti-Aβ antibody may be an anti-Aβ monoclonal antibody (mAb) selected from a group including bapineuzumab, solanezumab, aducanumab, gantenerumab, crenezumab, donanembab, and lecanemab. The patient may have suffered a side effect from the anti-Aβ antibody, such as brain edema or swelling (e.g., ARIA-E) and brain hemorrhaging or bleeding (e.g., ARIA-H).

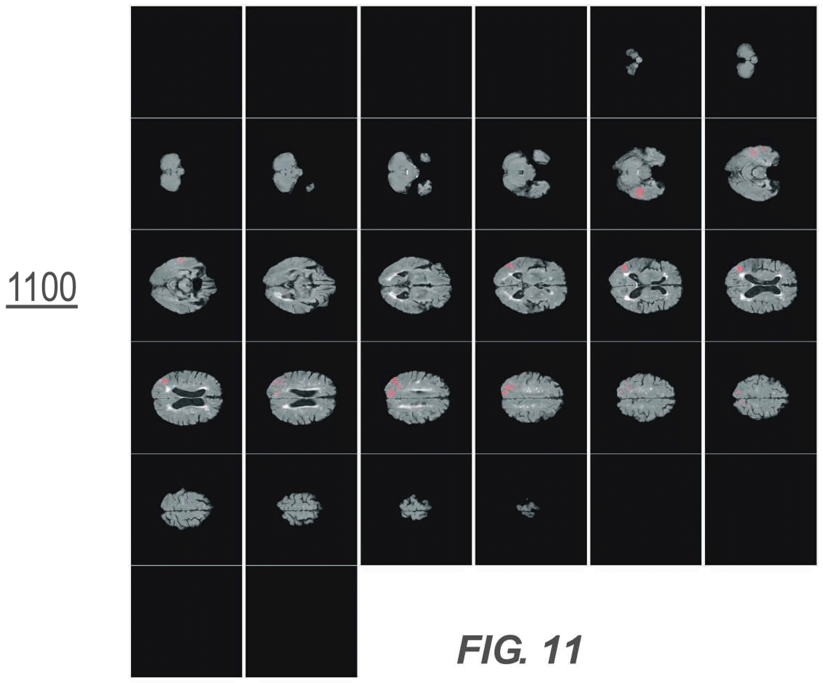

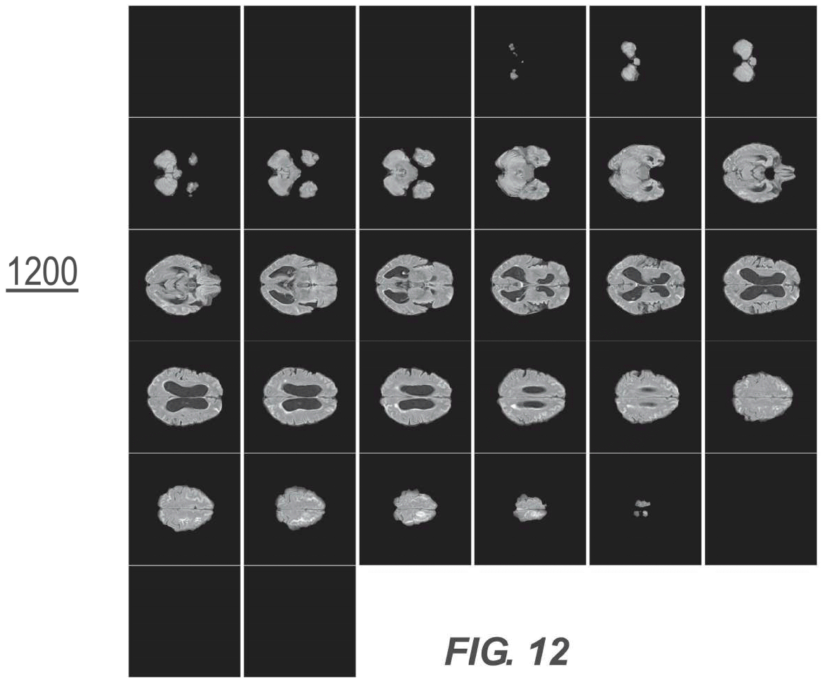

The set of one or more brain-scan images may include a plurality of medical images corresponding to a plurality of cross sections of a brain of the patient, as illustrated in FIGS. 11 and 12 described in more detail below. It should be appreciated that the one or more computing devices may receive other types of images other than MRI scans. In some embodiments, the set of one or more brain-scan images may include one or more MRI images, one or more positron emission tomography (PET) images, one or more single-photon emission computed tomography (SPECT) images, one or more amyloid PET images, or any combination thereof. The PET images may reveal the metabolic or biochemical function of tissues and organs, allowing the one or more computing devices to examine the functional components of the disease rather than structural components. The amyloid PET images may bring in more disease-specific information.

In some embodiments, the one or more computing devices may implement two different arms extracting images of different modalities and fuse the images using registration techniques. In some embodiments, the set of one or more brain-scan images may include one or more fluid-attenuated inversion recovery (FLAIR) images, one or more T2*-weighted imaging (T2*WI) images, one or more T1-weighted imaging (T1WI) images, or any combination thereof.

At block 304, the one or more computing devices may input the set of one or more brain-scan images into one or more machine-learning models (e.g., segmentation model 400) trained to generate a segmentation map (e.g., segmentation map 403) based on the set of one or more brain-scan images, in which the segmentation map (e.g., segmentation map 403) includes a plurality of pixel-wise class labels or voxel-wise class labels corresponding to a plurality of pixels or voxels in the segmentation map (e.g., segmentation map 403). The one or more machine-learning models (e.g., segmentation model 400) may generate one or more predicted probabilities corresponding to the plurality of pixel-wise class labels. In certain embodiments, at least one of the plurality of pixel-wise class labels or voxel-wise class labels may include an indication of ARIA in the brain of the patient.

For example, in one embodiment, for an input brain-scan image comprising *M*x*N* pixels arranged in a 2-dimentional (2D) grid, the segmentation model 400 may output a pixel-wise class label corresponding to each pixel of the *M*x*N* pixels in the input image. In another embodiment, the input brain-scan image may include a 3D volumetric scan including, for example, *M*x*N*x*P* voxels, and thus the segmentation model 400 may output a voxel-wise class label corresponding to each voxel of the *M*x*N*x*P* voxels in the input image. That is, the segmentation model 400 may either receive in 2D pixel data as described or 3D voxel data that may be arranged in a 3D grid or a stack corresponding to a subset of neighboring contiguous slices and/or cross-sectional volume of the patient's brain.

In certain embodiments, the semantic segmentation model 400 may include, for example, a semantic segmentation model, such as a full-resolution residual network (FRRN), a fully convolutional network (FCN) (e.g., U-Net, 3D U-Net), a harmonic dense neural network (HarDNet), a pyramid scene parsing network (PSPNet), a fully convolutional dense neural network (FCDenseNet), a multi-path refinement network (RefineNet), an atrous convolutional network (e.g., DeepLabV3, DeepLabV+), a semantic segmentation network (SegNet), or other similar semantic segmentation model suitable for generating a segmentation map 403 as to be described below with respect to FIG. 4.

At block 306, the one or more computing devices may output a quantification of ARIA in the brain of the patient based at least in part on the segmentation map. Thus, in certain embodiments, the segmentation model 400 may output a segmentation map (e.g., an image) in which the individual pixels or voxels corresponding to one or more *N* regions of interest with respect to the patient's brain, for example, are classified via binary class labels (e.g., "0" or "1" and/or "A State" or "B State") or multi-class class labels ("0", "1", . . ., "*N*" and/or "A State", "B State", . . . "*N* State"). That is, in accordance with the presently disclosed embodiments, each pixel or voxel within the segmentation map (e.g., a high-resolution image) may be labeled with a corresponding class label as a prediction of one or more ARIA lesions in the brain of the patient.

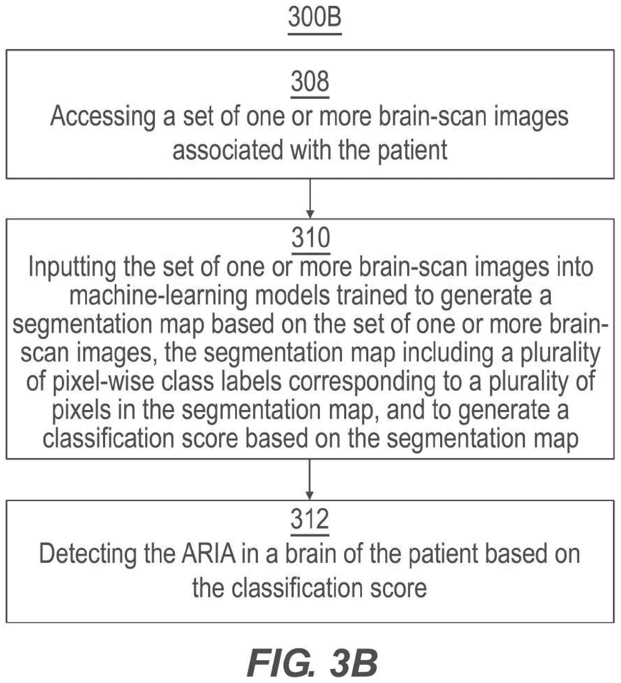

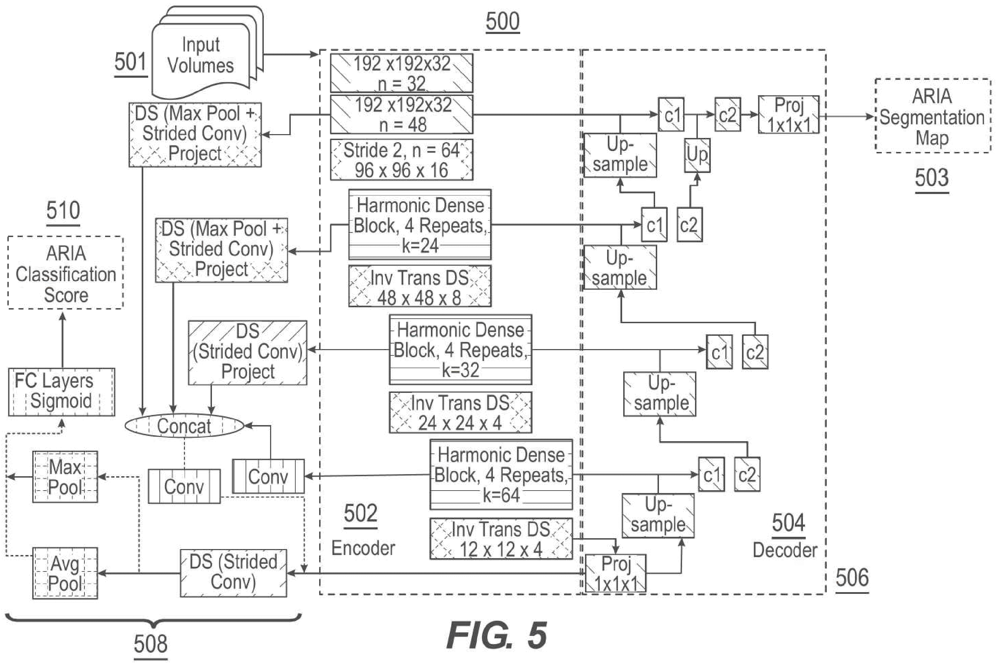

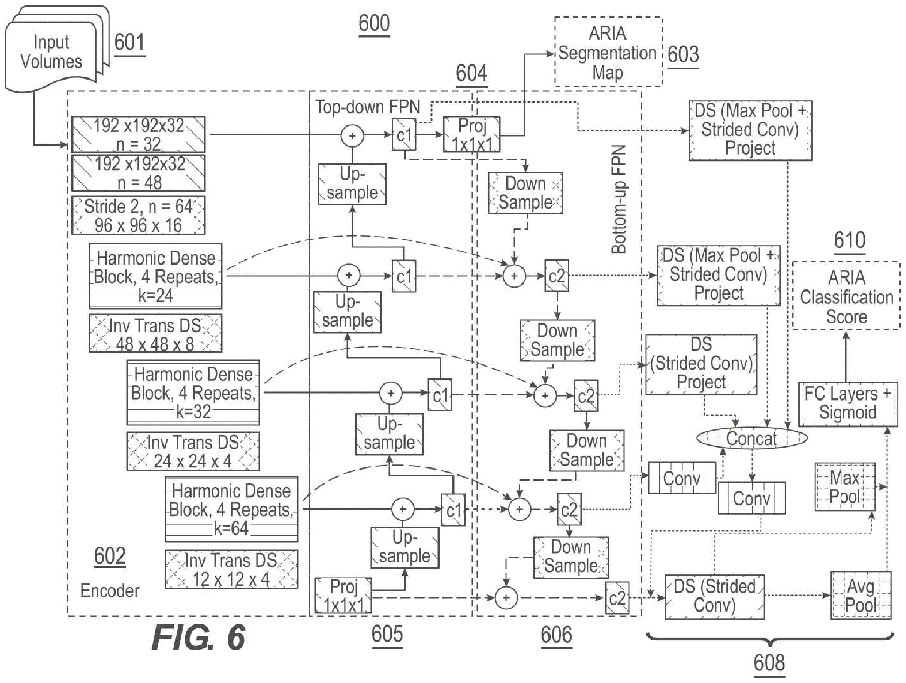

**FIG. 3B** illustrates an exemplary process 300B for detecting ARIA in the brain of a patient, according to various examples. In certain embodiments, the process 300B may be performed, for example, in accordance with the illustrated joint segmentation/classification model 500 to be discussed below with respect to FIG. 5 and/or joint segmentation/classification model 600 to be discussed below with respect to FIG. 6. The process 300B is performed, for example, using one or more electronic devices implementing a software platform. In some examples, process 300B is performed using a client-server system, and the blocks of process 300B are divided up in any manner between the server and one or more client devices. In other examples, process 300B is performed using only one or more client devices. In process 300B, some blocks are, optionally, combined, the order of some blocks is, optionally, changed, and some blocks are, optionally, omitted. In some examples, additional steps may be performed in combination with the process 300B. Accordingly, the operations as illustrated (and described in greater detail below) are exemplary by nature and, as such, should not be viewed as limiting.

The process 300B may be performed utilizing one or more processing devices (e.g., computing system and artificial intelligence architecture to be discussed below with respect to FIGS. 13 and 14) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing various medical data and making one or more decisions based thereon), software (e.g., instructions running/executing on one or more processors), firmware (e.g., microcode), or some combination thereof.

At block 308, an exemplary system including one or more computing devices may access a set of one or more brain-scan images associated with the patient. For example, as previously noted above, set of one or more brain-scan images may include a plurality of medical images corresponding to a plurality of cross sections of a brain of the patient, as illustrated in FIGS. 11 and 12 described in more detail below. It should be appreciated that the one or more computing devices may receive other types of images other than MRI scans. In some embodiments, the set of one or more brain-scan images may include one or more MRI images, one or more PET images, one or more SPECT images, one or more amyloid PET images, or any combination thereof. The PET images may reveal the metabolic or biochemical function of tissues and organs, allowing the one or more computing devices to examine the functional components of the disease rather than structural components. The amyloid PET images may bring in more disease-specific information.

In some embodiments, the one or more computing devices may implement two different arms extracting images of different modalities and fuse the images using registration techniques. In some embodiments, the set of one or more brain-scan images may include one or more FLAIR images, one or more T2*WI images, one or more T1WI images, or any combination thereof.

At block 310, an exemplary system including one or more computing devices may input the set of one or more brain-scan images into one or more machine-learning models (e.g., joint segmentation/classification model 500 and/or joint segmentation/classification model 600) trained to generate a segmentation map based on the set of one or more brain-scan images, in which the segmentation map includes a plurality of pixel-wise class labels corresponding to a plurality of pixels in the segmentation map, and to generate a classification score. In other embodiments, the one or more machine-learning models (e.g., joint segmentation/classification model 500 and/or joint segmentation/classification model 600) may generate one or more predicted probabilities corresponding to the plurality of pixel-wise class labels.

For example, as generally discussed above, the one or more machine-learning models may include a segmentation model 506 and classification model 508. For example, in certain embodiments, as generally discussed above, the segmentation model 400 of FIG. 4 or the segmentation model 506 may include, for example, one or more semantic segmentation models, such as an FRRN, an FCN (e.g., U-Net, 3D U-Net), HarDNet, PSPNet, an FCDenseNet, RefineNet, an atrous convolutional network (e.g., DeepLabV3, DeepLabV+), SegNet, or other similar semantic segmentation model that may be suitable for generating a segmentation map 503, in which one or more pixel-wise class labels or voxel-wise class labels may include an indication of ARIA in the brain of the patient. In certain embodiments, the classification model 508 may include, for example, one or more convolutional neural networks (CNNs), a deep neural network (DNN), a fully-connected neural network (FCNN), a residual neural network (ResNet) (e.g., ResNet-18, ResNet-34, ResNet-50, ResNet-101, ResNet-152, and so forth), or other similar neural network classification model that may be suitable for generating one or more classification scores 510 as to be described below with respect to FIG. 5, for example.

In certain embodiments, each pixel-wise class label or voxel-wise class label may be indicative of a measure related to ARIA. In some embodiments, a measure related to ARIA may be a binary value indicative of the presence of ARIA or the absence of ARIA (e.g., for the corresponding pixel or voxel in the input image and/or input volume). For example, in some embodiments, a binary value of "0" may indicate an absence of ARIA for a corresponding pixel or voxel in the input image and/or input volume, while a binary value of "1" may indicate the presence of ARIA for a corresponding pixel or voxel in the input image and/or input volume.

In some embodiments, a measure related to severity of ARIA may be assessed over the brain scan of the patient acquired during a patient visit or clinical trial and may include a numeric value (e.g., an integer value, a float value) indicative of the severity of ARIA over the 3D volume and/or the whole brain of the patient. For example, in some embodiments, a numeric value ranging from "0" to "10" may indicate varying levels of severity of ARIA. In some embodiments, the numeric value may be based on a scoring mechanism that has been developed to quantify ARIA. For example, a first exemplary scoring mechanism that has been developed to quantify ARIA is the Barkhof Grand Total Score (BGTS).

The BGTS score is based on twelve sub-scores corresponding to twelve bilateral regions of interest for ARIA-E (e.g., frontal right, frontal left, parietal right, parietal left, occipital right, occipital left, temporal right, temporal left, central right, central left, infratentorial right, infratentorial left). Each sub-score is a numeric value ranging from "0" to "5" representing the severity of ARIA-E, thus resulting in a total score ranging from "0" to "60." Additional information related to the BGTS scoring mechanism may be found in, for example, F. Barkhof, et al., "An MRI Rating Scale for Amyloid-Related Imaging Abnormalities with Edema or Effusion," American Journal of Neuroradiology August 2013, 34 (8) 1550-1555, the content of which is incorporated herein by reference.

Other exemplary scoring mechanisms include a simplified 3-point severity score and a simplified 5-point severity score. For example, the simplified 3-point severity score uses "0" to indicate absence of ARIA, "1" to indicate mild ARIA, "2" to indicate moderate ARIA; and "3" to indicate severe ARIA. Additional information related to the simplified scoring mechanisms may be found in, for example, L. Bracoud et al., "Validation of a Simple Severity Scale for Assessing ARIA-E," Alzheimer's & dementia: the journal of the Alzheimer's Association 13(7):P253-P254, the content of which is incorporated herein by reference. Further, correlations between 3- and 5-point scores and the BGTS score are studied, for example, in G. Klein et al., "Calibration of a Simplified ARIA-E MRI Severity Scale Suitable for Clinical Practice," Alzheimer's & Dementia December 2020, Volume 16, Issue S2.

At block 312, the one or more computing devices may then detect ARIA in the brain of the patient based on the classification score. For example, the classification score may be derived from the volume and spatial distribution of ARIA lesions delineated by the segmentation model 400. For example, a binary value of "0" may indicate an absence of ARIA in the AD patient corresponding to an absence of ARIA lesions in the predictions of the segmentation model 400 or the predicted volume being lower than a predefined threshold determined empirically. Similarly, a binary value of "1" may indicate the presence of ARIA in the AD patient. As another example, a binary value of "0" may indicate mild ARIA (e.g., "0" or "1" in the simplified 3-point scoring mechanism), while a binary value of "1" may indicate severe ARIA (e.g., "2" or "3" in the simplified 3-point scoring mechanism).

In some embodiments, the classification score for the classification task may be based on a scoring mechanism that has been developed to quantify ARIA, such as the simplified 3-point score, the simplified 5-point score, etc., thus converting it to a multiclass classification. In some embodiments, a regression model rather than a classification model may be used in block 312, for example to predict BGTS score. The regression result may include a numeric value (e.g., an integer value, a float value) indicative of the severity of ARIA (e.g., for the entire set of one or more images). For example, a numeric value ranging from "0" to "10" may indicate varying levels of severity of ARIA in the patient. It should be appreciated that, in some embodiments, as opposed to the joint segmentation/classification model 500 and/or joint segmentation/classification model 600 generating the classification score, the classification score may be manually assigned to the segmentation map 503, for example, by one or more clinicians (e.g., neurologists, radiologists, neurosurgeons) during or succeeding one or more patient visits or clinical trials.

**FIG. 4** illustrates an exemplary segmentation model 400 that may be used in block 304 to receive a set of one or more brain-scan images (e.g., input volumes 401) and in block 306 to generate a plurality of pixel-wise or voxel-wise class labels included as part of a segmentation map 403, in accordance with some embodiments. In the depicted example, the segmentation model 400 may include a trained encoder 402 and a trained decoder 404, as described in detail below.

In certain embodiments, the trained encoder 402 may be configured to receive a set of one or more images and obtain a plurality of down-sampled feature maps based on the received set of one or more images. In some embodiments, the encoder 402 may be a neural network, such as a harmonic dense neural network (HarDNet). In the depicted example in FIG. 4, the neural network is configured to receive as input a volume of 224x224x32. For example, in some embodiments, the input volumes 401 may include a dataset of 3D volumetric scans of one or more sections of the patient's brain. The neural network may include a group of layers called harmonic dense blocks and each harmonic dense block may be followed by an inverted transition down-sampling block (illustrated as "Inv Trans DS" in FIG. 4) to produce a plurality of down-sampled feature maps. Additional information related to the HarDNet may be found, for example, in P. Chao et al., "HarDNet: A Low Memory Traffic Network," 2019 IEEE/CVF International Conference on Computer Vision (ICCV), the content of which is incorporated herein by reference.

In certain embodiments, the trained decoder 404 may be configured to generate the pixel-wise or voxel-wise class labels included as part of a segmentation map 403 (e.g., a pixel-wise or voxel-wise annotated image) based on the plurality of down-sampled feature maps outputted by the encoder 402. In some embodiments, the decoder 404 may be a neural network, such as a U-Net decoder. In the depicted example in FIG. 4, the neural network may include a plurality of convolution blocks (illustrated as "c1" and "c2" in FIG. 4) that receive the down-sampled feature maps from the trained encoder 402 and output the segmentation map 403.

Specifically, in certain embodiments, the trained encoder 402 may include the "contraction" stage of the segmentation model 400. The "contraction" stage of the segmentation model 400 may include the section of the segmentation model 400 utilized to generate the down-sampled feature maps based on the input volumes 401. Similarly, in certain embodiments, the trained decoder 404 may include the "expansion" stage of the segmentation model 400. The "expansion" stage of the segmentation model 400 may include the section of the segmentation model 400 utilized to generate a number of up-sampled feature maps based on features learned through the down-sampling performed by the trained encoder 402, such that the trained decoder 404 generates a segmentation map 403 (e.g., a pixel-wise or voxel-wise annotated image) that corresponds generally to the input volumes 401.

In certain embodiments, the segmentation model 400 may provide a number of technical advantages. For example, the implementation of the segmentation model 400 may be memory-efficient because the segmentation model 400 may be able to fit the entire 3D input volumes 401 as the input to the segmentation model 400. This may allow the segmentation model 400 to be easily fine-tuned for downstream tasks (e.g., classification, regression), as described below. Further, the segmentation model 400 may enable easy flow of information from local size scale to global size scale, thus providing more accurate segmentation results because ARIA information may be generally local and relatively small in size. Further, the segmentation model 400 may include a relatively more intensive encoder and a relatively less intensive decoder, such that decoding may be performed efficiently. For at least the reasons above, the design and implementation of the segmentation model 400 may improve the functioning of a computer by requiring less memory, processing power, and power consumption.

It should be appreciated that the segmentation model 400 depicted in FIG. 4 is merely exemplary and that other models, such as other machine-learning models, may be used to process brain-scans to obtain the pixel-wise or voxel-wise labels as described above. Indeed, in some instances, in which fitting the entire 3D volume into GPU memory or other storage resources may not be feasible, the training of the segmentation model 400 may be performed on images patches or 2D tiles (e.g., utilizing multiple-instance learning (MIL)) obtained by dividing the 3D volume into smaller 2D / 3D blocks that may or may not overlap.

In certain embodiments, the training of the segmentation model 400 used in blocks 304 and 306 of the process 300A of FIG. 3A may be performed using a number of approaches. In some embodiments, the segmentation model 400 may be trained utilizing, for example, MRI datasets of patients with ARIA and an equal number of samples or approximately equal number of samples from AD patients without ARIA. As ARIA is an adverse event and has lower prevalence, the training datasets may be a smaller than would what otherwise be desirable for training a deep neural network (DNN). Thus, in some embodiments, the segmentation model 400 may be trained utilizing a transfer learning process, for example, adapted from a different segmentation model.

For example, in some embodiments, the segmentation model 400 for identifying ARIA used in blocks 304 and 306 of the process 300A of FIG. 3A may be fine-tuned from a different segmentation model trained to identify Multiple Sclerosis (MS) lesions in medical images. In some embodiments, during transfer learning, for example, the entire segmentation model 400, including the encoder 402 and the decoder 404, may be trained together at a reduced learning rate. In some embodiments, the decoder 404 may be trained first while the encoder 402 remains fixed, and the one or more computing devices on which the segmentation model 400 is executed may progressively unlock training of layers in the encoder 402 starting from the deepest layer to the shallowest layers. The progressive training approach may be beneficial because the deeper layers may be more domain-specific and thus may be advantageously trained first.

In certain embodiments, to reduce over-fitting, the segmentation model 400 may be trained with image augmentations (e.g., rotations, translations, and scaling) and/or affine transformations and elastic deformations. Additionally, the segmentation model 400 may utilize drop out during training and MixUp regularization, which is a data augmentation technique that creates new training data inputs and targets as combinations of samples from the training dataset. The segmentation model 400 may be trained with *n*-fold cross-validation or nested cross-validation using a combined dice loss and weighted binary cross entropy loss terms. In certain embodiments, the segmentation model 400 may use multimodal inputs from various MRI sequences with the slices and/or patches stacked along the channel dimension or MRI and PET volumes with features from each input extracted using a separate arm of the segmentation model 400 and combined by addition or concatenation to be used as skip features for the decoder 404.

In response to detecting ARIA in the brain of the patient, the one or more computing devices may determine a dosage adjustment of the anti-Aβ antibody. In some embodiments, if ARIA is detected, the one or more computing devices may recommend a reduced dosage of the anti-Aβ antibody. The recommendation may be provided via one or more outputs (e.g., visual, auditory, haptic outputs), by generating a report for a clinician, etc.

In some embodiments, if ARIA is detected, the one or more computing devices may determine a reduced dosage of the anti-Aβ antibody. For example, the one or more computing devices can compare the results of the models to one or more predefined thresholds to determine the severity of ARIA. In accordance with a determination that the detected ARIA is mild, the system can determine a first reduced dosage. In accordance with a determination that the detected ARIA is severe, the one or more computing devices can determine a second reduced dosage lower than the first reduced dosage. The determined dosage may be provided via one or more outputs (e.g., visual, auditory, haptic outputs), by generating a report for a clinician, etc. In some embodiments, the one or more computing devices may automatically control a medical device to administer the reduced dosage of the anti-Aβ antibody to the patient.

In some embodiments, if ARIA is detected, the one or more computing devices may determine to terminate or temporarily suspend the prescription or administration of the anti-Aβ antibody to the patient. For example, the one or more computing devices can compare results of the models to one or more predefined criteria to determine if the anti-Aβ antibody should be terminated or temporarily suspended. The termination or suspension decision may be provided via one or more outputs (e.g., visual, auditory, haptic outputs), by generating a report for a clinician, etc. In some embodiments, the one or more computing devices may automatically control a medical device to terminate or temporarily suspend the administration of the anti-Aβ antibody to the patient.

In some embodiments, if ARIA is detected, the one or more computing devices may determine one or more anti-ARIA treatments (e.g., one or more anti-ARIA antibodies) for the patient. For example, the one or more computing devices can compare results of the models to one or more predefined thresholds to determine the recommended treatment. The identified treatments may be provided via one or more outputs (e.g., visual, auditory, haptic outputs), by generating a report for a clinician, etc. In some embodiments, the one or more computing devices may automatically control a medical device to administer the anti-ARIA treatments to the patient.

The one or more computing devices may monitor ARIA in a patient over time. In some embodiments, the one or more computing devices may be configured to receive different sets of medical images corresponding to different time points and analyze the images using the techniques described herein. By monitoring ARIA in the patient over time, the one or more computing devices can determine whether any of the responses above (e.g., reduced dosage, terminated or temporarily suspended administration, anti-ARIA treatments) is effective, and formulate an adjusted response accordingly. In some embodiments, the one or more computing devices may monitor ARIA in multiple patients that have received different types of anti-Aβ antibodies and, by comparing the presence and/or severity of ARIA in these patients over time, determine the safeness of these different types of anti-Aβ antibodies to inform future treatment decisions.

**FIG. 5** depicts an exemplary joint segmentation/classification model 500 that may be used in blocks 310 and 312 of the process 300B of FIG. 3B, in accordance with some embodiments. With reference to FIG. 5, the joint segmentation/classification model 500 may include a classification model 508 (e.g., classification decoder) that is configured to operate in conjunction with a segmentation model 506, and the segmentation model 506 includes a segmentation encoder 502 and a segmentation decoder 504. In certain embodiments, the segmentation encoder 502 may include, for example, a HarDNet encoder. In certain embodiments, the segmentation decoder 504 may include, for example, a U-Net decoder. In certain embodiments, the classification model 508 may include, for example, one or more CNNs, a DNN, an FCNN, a residual neural network (ResNet) (e.g., ResNet-18, ResNet-34, ResNet-50, ResNet-101, ResNet-152, and so forth), or other similar neural network classification model that may be suitable for generating one or more classification scores.

In the depicted example, the segmentation model 506 may be identical or similar to the segmentation model 400 in FIG. 4, with the segmentation encoder 502 being identical or similar to the encoder 402 and the segmentation decoder 504 being identical or similar to the decoder 404. During training, the joint segmentation/classification model 500 weights are optimized for both segmentation tasks (e.g., segmenting the input volumes 501 on a pixel-by-pixel basis or voxel-by-voxel basis to generate an output annotated segmentation map 503) and one or more classification scores 510 (e.g., one or more probabilities or scores classifying a presence or absence of ARIA and/or severity of ARIA) by minimizing a combination of segmentation and classification loss terms.

In certain embodiments, the joint segmentation/classification model 500 may be trained or implemented in accordance with a multi-task learning process, which improves segmentation model generalizability. For example, the ARIA lesions (e.g., areas of diffuse swelling), for example, may be challenging for the segmentation model 506, and thus the classification model 508 may provide an additional mechanism for predicting ARIA scores, which may, in some embodiments, be complementary to the ARIA scores generated based on the segmentation map 503 predicted by the segmentation model 506.

The segmentation encoder 502 may be configured to obtain a plurality of down-sampled feature maps based on a set of one or more brain-scan images (e.g., input volumes 501) associated with the patient, as described above with reference to FIG. 4. Further, with reference to FIG. 5, the classification decoder 504 may be configured to generate one or more classification scores 510 (e.g., one or more scores or probabilities for classifying a presence or absence of ARIA and/or severity of ARIA) based on down-sampled feature maps obtained from the layers (e.g., harmonic dense blocks) of the segmentation encoder 502.

In some embodiments, the classification score 510 may be one or more scores generated by a sigmoid layer based on the embeddings in the fully connected layers learned and estimated from the down-sampled feature maps obtained from the layers in the encoder 502 (e.g., harmonic dense blocks). In the depicted example, the down-sampled feature maps are obtained and aggregated from multiple layers corresponding to varying resolution and/or scale of features of the segmentation encoder 502. As opposed to using features from the deepest convolution layer only, this implementation may be particularly advantageous because it may ensure that both global and local information may be captured.

**FIG. 6** depicts another exemplary joint segmentation/classification model 600 that may be used in blocks 310 and 312 of the process 300B, in accordance with some embodiments. In the depicted example, the joint segmentation/classification model 600 may include a segmentation encoder 602, a segmentation decoder 604 (e.g., FPN 605), and a classification model 608 (e.g., classification decoder). A bidirectional feature propagation network including a top-down feature pyramid network (FPN) 605 and a bottom-up FPN 606 may be used for the segmentation decoder 604 and the classification model 608. In the depicted example, the top-down FPN 605 may be identical or similar to the decoders 404 and 504 and may be configured to output the segmentation map 603 based on, for example, a series of feature maps corresponding to the input volumes 601.

In certain embodiments, the use of a bidirectional feature propagation network may be technically advantageous because the features extracted by the segmentation encoder 602 and corresponding to the generated segmentation map 603 are combined optimally with segmentation relevant features extracted by the top-down FPN 605 in the bottom-up FPN 606 to be used as features for the classification task (e.g., generating one or more probabilities or scores 610 for classifying a presence or absence of ARIA and/or severity of ARIA). The classification model 608 (e.g., classification decoder) may be configured to receive input data from the layers of the bottom-up FPN 606 to generate the classification score 610. In one embodiment, the classification score 610 may be one or more scores generated by a sigmoid layer based on the learned embeddings in the fully connected layers from the down-sampled feature maps obtained from the layers of the bottom-up FPN 606.

In certain embodiments, the training of the joint segmentation/classification model (e.g., models 500 and 600) may be performed in multiple stages. In the first stage, the model (e.g., models 500 and 600) may be pre-trained on the segmentation task (e.g., segmenting the input volumes 601 on a pixel-by-pixel basis or voxel-by-voxel basis to generate an output annotated segmentation map 603). For example, in the first stage, one or more training images (e.g., input volumes 601) may be provided to segmentation portion of the model (e.g., encoder 502 and decoder 504 of model 500; encoder 602 and decoder 604 of model 600) to train the segmentation task, while the classification portion of the model (e.g., classification model 508 of model 500; bottom up FPN 606 and classification model 608 of model 600) remain fixed.

In certain embodiments, during training, the weights of the segmentation portion of the model (e.g., encoder 502 and decoder 504 of model 500; encoder 602 and decoder 604 of model 600) may be updated by comparing the segmentation outputs and the ground truth labels of the training images (e.g., via a backpropagation process), while the weights of the classification portion of the model (e.g., classification model 508 of model 500; bottom up FPN 606 and classification model 608 of model 600) remain fixed. In the second stage, the entire model (e.g., models 500 and 600) or only the classification portion of the model (e.g., classification model 508 of model 500; bottom up FPN 606 and classification model 608 of model 600) may be trained to perform the classification task.

For example, in the second stage, the weights of the classification portion of the model (e.g., classification model 508 of model 500; bottom up FPN 606 and classification model 608 of model 600) may be updated by comparing the classification outputs and the ground truth labels of the training images (e.g., via a backpropagation process), while the weights of the segmentation portion of the model (e.g., encoder 502 and decoder 504 of model 500; encoder 602 and decoder 604 of model 600) may or may not remain fixed.

In certain embodiments, in response to detecting ARIA in the brain of the patient, the one or more computing devices may determine a dosage adjustment of the anti-Aβ antibody. In some embodiments, if ARIA is detected, the one or more computing devices may recommend a reduced dosage of the anti-Aβ antibody. The recommendation may be provided via one or more outputs (e.g., visual, auditory, haptic outputs) by generating a report for a clinician, etc.

In some embodiments, if ARIA is detected, the one or more computing devices may determine a reduced dosage of the anti-Aβ antibody. For example, the one or more computing devices may compare the results of the model (e.g., models 500 and 600) to one or more predefined thresholds to determine the severity of ARIA. In accordance with a determination that the detected ARIA is mild, the one or more computing devices may determine a first reduced dosage. In accordance with a determination that the detected ARIA is severe, the one or more computing devices may determine a second reduced dosage lower than the first reduced dosage. The determined dosage may be provided via one or more outputs (e.g., visual, auditory, haptic outputs) by generating a report for a clinician, etc. In some embodiments, the one or more computing devices may automatically control a medical device to administer the reduced dosage of the anti-Aβ antibody to the patient.

In some embodiments, if ARIA is detected, the one or more computing devices may determine to terminate or temporarily suspend the prescription or administration of the anti-Aβ antibody to the patient. For example, the one or more computing devices may compare results of the models to one or more predefined criteria to determine if the anti-Aβ antibody should be terminated or temporarily suspended. The termination or suspension decision may be provided via one or more outputs (e.g., visual, auditory, haptic outputs), by generating a report for a clinician, etc. In some embodiments, the one or more computing devices may automatically control a medical device to terminate or temporarily suspend the administration of the anti-Aβ antibody to the patient.

In some embodiments, if ARIA is detected, the one or more computing devices may determine one or more anti-ARIA treatments (e.g., one or more anti-ARIA antibodies) for the patient. For example, the one or more computing devices may compare results of the model (e.g., models 500 and 600) to one or more predefined thresholds to determine the recommended treatment. The identified treatments may be provided via one or more outputs (e.g., visual, auditory, haptic outputs) by generating a report for a clinician, etc. In some embodiments, the one or more computing devices may automatically control a medical device to administer the anti-ARIA treatments to the patient.

The one or more computing devices may monitor ARIA in a patient over time. In some embodiments, the one or more computing devices may be configured to receive different sets of medical images corresponding to different time points and analyze the images using the techniques described herein. By monitoring ARIA in the patient over time, the one or more computing devices may determine whether any of the responses above (e.g., reduced dosage, terminated or temporarily suspended administration, anti-ARIA treatments) is effective, and formulate an adjusted response accordingly. In some embodiments, the one or more computing devices may monitor ARIA in multiple patients that have received different types of anti-Aβ antibodies and, by comparing the presence and/or severity of ARIA in these patients over time, determine the safeness of these different types of anti-Aβ antibodies to inform future treatment decisions.

Accordingly, as generally described with respect to the joint segmentation/classification model 500 of FIG. 5 and/or the joint segmentation/classification model 600 of FIG. 6, the present embodiments may provide techniques to accurately segment and classify brain scans (e.g., MRI scans, PET scans) for segmenting, detecting, and quantifying ARIA, which may manifest as contextual changes and/or changes in signal intensities in the brain scans (e.g., MRI scans, PET scans). Specifically, as illustrated with respect to the joint segmentation/classification model 500 and/or the joint segmentation/classification model 600, the present embodiments may further provide techniques to train and utilize a joint segmentation and classification model to accurately segment and classify brain scans (e.g., MRI scans, PET scans) for predicting, detecting, and quantifying ARIA.

However, in some embodiments, accurately training the joint segmentation/classification model 500 and/or the joint segmentation/classification model 600 may rely on training data (e.g., ground truth data of both pixel-wise or voxel-wise annotated images and 3D volume ARIA scoring) that may not be readily available (e.g., as ARIA may be observed clinically in only a small subgroup of AD patients of a much larger group of AD patients having been treated utilizing anti-Aβ mAbs) and/or that may require excessive and costly image annotations or volume annotations to be performed manually by human annotators.

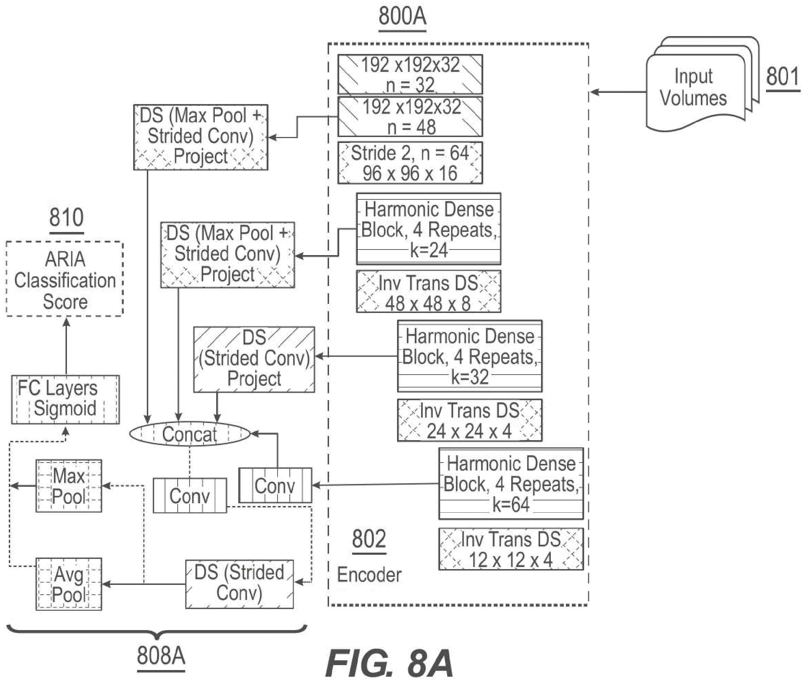

Thus, in certain embodiments, to overcome the limited training data, the present embodiments may provide techniques to train and utilize a distinct segmentation model (e.g., segmentation model 400 as described above with respect to FIG. 4) to segment brain scans (e.g., input volumes 401, input volumes 801) to identify ARIA lesions in the brain of the patient, and another distinct classification model (e.g., classification model 800A as to be described in greater detail below with respect to FIG. 8A) to classify the segmented brain scans (e.g., input volumes 401, input volumes 801) by predicting ARIA scores corresponding to a presence or severity of the identified ARIA lesions in the brain of the patient. That is, in contrast to the joint segmentation/classification model 500 of FIG. 5 and/or the joint segmentation/classification model 600 of FIG. 6, the segmentation tasks (e.g., semantic segmentation tasks) and classification tasks may be divided across separate and distinct segmentation (e.g., segmentation model 400 as described above with respect to FIG. 4) and classification (e.g., classification model 800A as to be described in greater detail below with respect to FIG. 8A) models.

For example, in some embodiments, the segmentation model 400 and the classification model 800A (as described in greater detail below with respect to FIG. 8A) may be separately trained for the respective segmentation and classification tasks, where the output (e.g., segmentation map 403 or one or more predicted probabilities) of the segmentation model 400 may provide areas or features to which the classification model 800A is to pay attention. Such a disjoint approach may not require that all the training samples include both manual pixel-wise or voxel-wise annotations for training the segmentation model 400 for the segmentation task and volume-level classification and/or regression class labels for training the classification model 800A for the classification task. Indeed, as previously noted, in accordance with the presently disclosed techniques, in some embodiments, the segmentation model 400 and the classification model 800A may be accurately and separately trained to perform the disclosed segmentation tasks and classification tasks utilizing only a limited training dataset (e.g., as ARIA may be observed clinically in only a small subgroup of AD patients of a much larger group of AD patients having been treated utilizing anti-Aβ mAbs).

In certain embodiments, the segmentation model 400 may be trained prior to separately training the classification model 800A. In certain embodiments, a set of weights may be learned during the training of the segmentation model 400. Subsequent to training the segmentation model 400 and learning the set of weights, the encoder 802 of the classification model 800A may be initialized with the set of weights learned from the training of the segmentation encoder 402 of the segmentation model 400. As previously noted, in certain embodiments, the segmentation model 400 may generate one or more predicted probabilities corresponding to a plurality of pixel-wise or voxel-wise class labels indicative of ARIA. For example, in some embodiments, the pixel-wise or voxel-wise predicted probabilities for ARIA generated by the segmentation model 400 may be then used as an additional input to the classification model 800A, or may be used to modulate the feature maps extracted by the classification encoder 802 of the classification model 800A.

In certain embodiments, the classification model 800A may include an attention mechanism to enhance at least some portions of the input volumes 801 while diminishing other portions of the input volumes 801. Such a technique thus emphasizes the most important portion of the input volumes 801. In some embodiments, the attention mechanism may be configured to focus on areas (e.g., pixels or voxels) or features in the input volumes 801 that are indicative of the presence of ARIA or absence of ARIA and/or severity of ARIA. For example, the attention mechanism may be based on the pixel-wise or voxel-wise predicted probabilities generated by the segmentation model 400.

In some embodiments, the attention mechanism may be configured to focus on areas (e.g., pixels or voxels) or features in the input volumes 801 that depict dilated grey matter to provide attention to brain surfaces or folds. For example, the attention mechanism may be based on dilated gray matter segmentation labels or masks, which may be part of the input volumes 801 or may be provided by a separate machine-learning model, for example. In some embodiments, the attention mechanism may be configured to focus on areas (e.g., pixels or voxels) or features in the input volumes 801 that have changed over time. For example, the attention mechanism may be based on subtraction labels or masks. The subtraction labels or masks may be generated, for example, from T1WI images from baseline time point, in some embodiments.

In certain embodiments, for cases in which it is difficult fit an entire 3D volume as represented by the input volumes 801 into GPU memory or other storage, the training of the classification model 800A may be performed on 3D patches or 2D tiles. In such a case, the prediction for the 3D volume as represented by the input volumes 801 may be obtained using multiple-instance learning (MIL) techniques with mean-pooling, max-pooling, or weightedpooling of the predictions from the various 3D patches or 2D tiles corresponding to the 3D volume as represented by the input volumes 801.

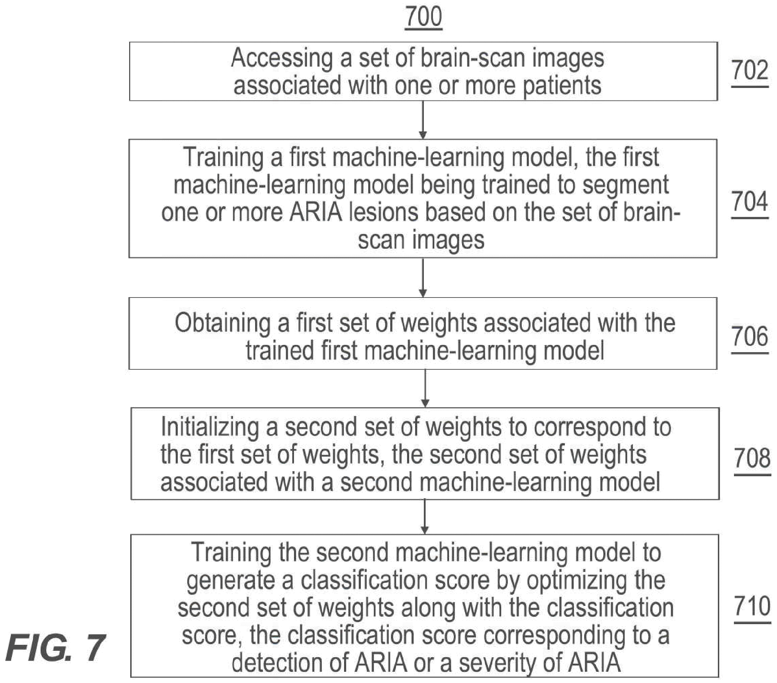

**FIG. 7** illustrates an exemplary process 700 for training a plurality of machine-learning models (e.g., segmentation model 400 and classification model 800A) for detecting ARIA in brains of patients, according to various examples. Process 700 is performed, for example, using one or more electronic devices implementing a software platform. In some examples, process 700 is performed using a client-server system, and the blocks of process 700 are divided up in any manner between the server and one or more client devices. In other examples, process 700 is performed using one or more client devices. In process 700, some blocks are, optionally, combined, the order of some blocks is, optionally, changed, and some blocks are, optionally, omitted. In some examples, additional steps may be performed in combination with the process 700. Accordingly, the operations as illustrated (and described in greater detail below) are exemplary by nature and, as such, should not be viewed as limiting.

The process 700 may be performed utilizing one or more processing devices (e.g., computing system and artificial intelligence architecture to be discussed below with respect to FIGS. 13 and 14) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing various medical data and making one or more decisions based thereon), software (e.g., instructions running/executing on one or more processors), firmware (e.g., microcode), or some combination thereof.

At block 702, an exemplary system including one or more computing devices may access a set of brain-scan images associated with one or more patients. The one or more computing devices may retrieve the set of brain-scan images from one or more computer memories, from one or more imaging devices, from one or more local or remote databases, or any other data sources. The one or more computing devices may access the set images automatically or in response to a user input. The set of brain-scan images may be taken before, during, or after a treatment is administered to the patient. In some embodiments, the patient is an Alzheimer's disease patient having been treated with an anti-Aβ antibody. The patient may have suffered a side effect from the anti-Aβ antibody, such as brain swelling (e.g., ARIA-E) and small brain bleeding (e.g., ARIA-H).

The set of brain-scan images may include a plurality of medical images corresponding to a plurality of cross sections of a brain of the patient as illustrated in FIGS. 11 and 12 described in detail below. It should be appreciated that the one or more computing devices may receive other types of images other than MRI scans. In some embodiments, the set of brain-scan images may include one or more MRI images, one or more positron emission tomography (PET) images, one or more single-photon emission computed tomography (SPECT) images, one or more amyloid PET images, or any combination thereof. The PET images may reveal the metabolic or biochemical function of tissues and organs, allowing the one or more computing devices to examine the functional components of the disease rather than structural components. The amyloid PET images may bring in more disease-specific information.

In some embodiments, the one or more computing devices may implement two different arms extracting images of different modalities and fuse the images using registration techniques. In some embodiments, the set of brain-scan images may include one or more fluid-attenuated inversion recovery (FLAIR) images, one or more T2*-weighted imaging (T2*WI) images, one or more T1-weighted imaging (T1WI) images, or any combination thereof.

At block 704, the one or more computing devices may then train a first machine-learning model (e.g., segmentation model 400) of the plurality of machine-learning models, in which the first machine-learning model (e.g., segmentation model 400) is trained to segment one or more ARIA lesions based on the set of brain-scan images. For example, in certain embodiments, the segmentation model 400 may receive the input volumes 401 and generate one or more predicted probabilities corresponding to a plurality of pixel-wise or voxel-wise class labels indicative of one or more ARIA lesions. In some embodiments, the pixel-wise or voxel-wise predicted probabilities labels for ARIA generated by the segmentation model 400 may be used as an additional input to the classification model 800A, or may be used to modulate the feature maps extracted by the classification encoder 802 of the classification model 800A, for example.

At block 706, the one or more computing devices may then obtain a first set of weights associated with the trained first machine-learning model (e.g., segmentation model 400). For example, in some embodiments, the segmentation model 400 and the classification model 800A may be trained in accordance with a transfer learning process, in which the segmentation model 400 may be trained prior to separately training the classification model 800A, and a set of weights may be learned during the training of the segmentation model 400. At block 708, the one or more computing devices may then initialize a second set of weights to correspond to the first set of weights, in which the second set of weights are associated with a second machine-learning model (e.g., classification model 800A). For example, in some embodiments, the encoder 802 of the classification model 800A may be initialized with the set of weights learned from the training of the segmentation encoder 402 of the segmentation model 400.

At block 710, the one or more computing devices may then train the second machine-learning model (e.g., classification model 800A) to generate a classification score based at least in part on the second set of weights,. , in which the classification score corresponds to a detection of a presence of ARIA or a severity of ARIA in the brains of one or more patients. For example, in certain embodiments, the classification model 800A may be trained based on the one or more predicted probabilities of ARIA corresponding to a plurality of pixel-wise or voxel-wise class labels indicative of one or more ARIA lesions generated by the segmentation model 400 and the set of weights learned during the training of the segmentation model 400 to generate one or more classification scores. For example, the one or more classification scores may be indicative of whether the one or more patients have ARIA and/or a severity of ARIA (e.g., mild ARIA, moderate ARIA, and severe ARIA).

In some embodiments, the one or more classification scores may be a value indicative of an ARIA class, for example, the presence or absence of ARIA (e.g., for the entire set of one or more images) in the one or more patients. For example, a binary value of "0" may indicate an absence of ARIA in the one or more patients, while a binary value of "1" may indicate the presence of ARIA in the one or more patients. As another example, a binary value of "0" may indicate mild ARIA (e.g., "0" or "1" in the simplified 3-point scoring mechanism), while a binary value of "1" may indicate severe ARIA (e.g., "2" or "3" in the simplified 3-point scoring mechanism).

In some embodiments, a regression model rather than a classification model 800A is used in block 710. The regression score may include a numeric value (e.g., an integer value, a float value) indicative of the severity of ARIA (e.g., for the entire set of one or more images). For example, a numeric value ranging from "0" to "10" may indicate varying levels of severity of ARIA in the one or more patients. In some embodiments, the numeric value may be based on a scoring mechanism that has been developed to quantify ARIA, such as the BGTS score, the simplified 3-point score, the simplified 5-point score, etc.

**FIG. 8A** illustrates an exemplary ARIA classification model 800A that may be used in the block 710, in accordance with some embodiments. The ARIA classification model 800A may include an encoder 802, which may receive one or more input volumes 801. In the depicted example in FIG. 8A, the ARIA classification model 800A may further include a classification model 808A. In certain embodiments, the encoder 802 may be a neural network, such as a HarDNet, and may be identical or similar to the encoder 402 described above with respect to FIG. 4, for example. In certain embodiments, the encoder 802 may generate a plurality of down-sampled feature maps based on the one or more input volumes 801. In certain embodiments, the encoder 802 may then pass the plurality of down-sampled feature maps to the classification model 808A. In some embodiments, the classification model 808A may then generate one or more classification scores 810 based on the plurality of down-sampled feature maps outputted by the encoder 802.

For example, in certain embodiments, the classification model 800A may generate one or more classification scores 810 that may be indicative of whether one or more patients have ARIA and/or a severity of ARIA (e.g., mild ARIA, moderate ARIA, and severe ARIA). In some embodiments, the one or more classification scores 810 may be a value indicative of an ARIA class, for example, the presence or absence of ARIA (e.g., for the entire set of one or more images) in the one or more patients. For example, a binary value of "0" may indicate an absence of ARIA in the one or more patients, while a binary value of "1" may indicate the presence of ARIA in the one or more patients. As another example, a binary value of "0" may indicate mild ARIA (e.g., "0" or "1" in the simplified 3-point scoring mechanism), while a binary value of "1" may indicate severe ARIA (e.g., "2" or "3" in the simplified 3-point scoring mechanism).

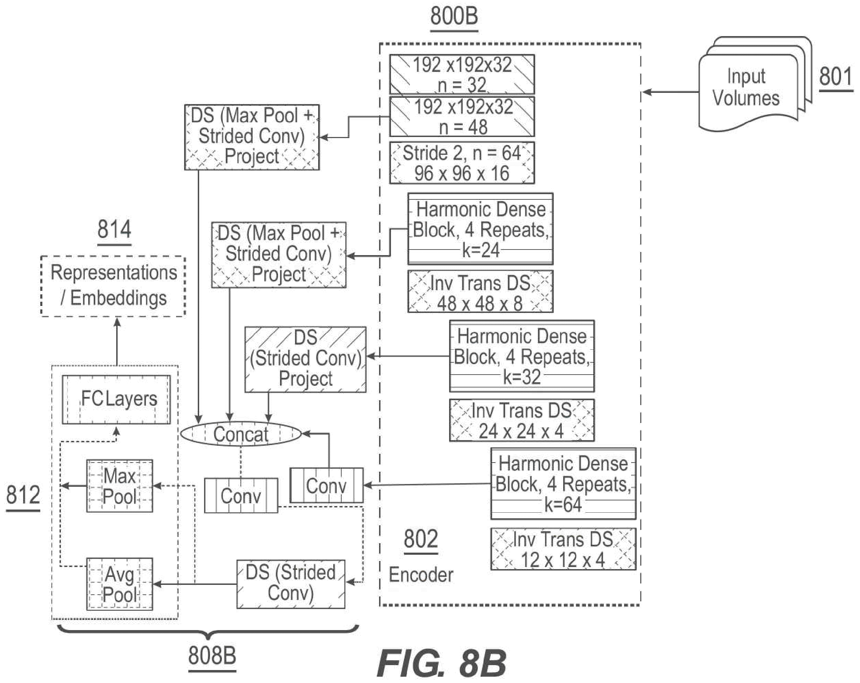

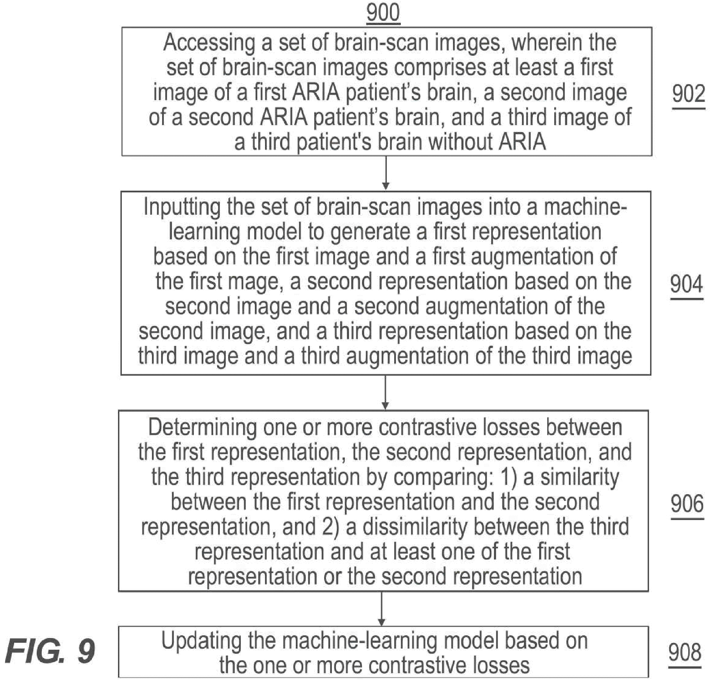

**FIG. 8B** illustrates an exemplary pre-training classification model 800B, in accordance with some embodiments. In certain embodiments, the pre-training classification model 800B may be pre-trained in accordance with a classification pre-training process 900 to be discussed below with respect to FIG. 9. As used herein, "pre-training" may refer to a machine-learning training process (e.g., by way of unsupervised learning, weakly-supervised learning, semisupervised learning, or self-supervised learning) that may be performed, for example, prior to training the machine-learning model(s) for tasks, such as segmentation, classification, regression, and so forth. In certain embodiments, the pre-training classification model 800B may include the encoder 802 and a pre-training classification model 808B that may be similar to the classification model 808A as discussed above with respect to FIG. 8A. For example, in some embodiments, the pre-training classification model 808B may include, for example, one or more CNNs or one or more residual neural networks (ResNet) (e.g., ResNet-18, ResNet-34, ResNet-50, ResNet-101, ResNet-152, and so forth), or other similar neural network classification model that may be suitable for generating one or more representations or embeddings 814.

In the depicted example in FIG. 8B, the pre-training classification model 808B may include a pre-training classification arm 812, which may include one or more average pooling layers, one or more maximum pooling layers, block, and one or more fully connected layers. In certain embodiments, the pre-training classification model 808B may be pre-trained utilizing one or more contrastive learning processes (e.g., supervised contrastive learning, self-supervised contrastive learning) and/or other similar self-supervised learning (SSL) techniques. For example, in some embodiments, the one or more contrastive learning processes may include, for example, any of a number of machine-learning processes (e.g., data augmentation, translation or encoding, contrastive loss function minimization, and so forth) utilized in conjunction to teach the pre-training classification model 808B to learn meaningful, high-level representations or embeddings 814 of the input volumes 801 without any use of, or with only limited use of, class labeled or annotated training data sets. For example, the pre-training classification model 808B may learn meaningful, high-level representations or embeddings 814 without any use of, or with only limited use of, class labeled or annotated training data by learning to determine similarity and/or dissimilarity between the representations or embeddings 814.

Specifically, as part of the contrastive learning (e.g., supervised contrastive learning, self-supervised contrastive learning) pre-training of the pre-training classification model 808B, all layers of the pre-training classification model 808B with the exception of the last fully connected layers and sigmoid layers of the classification arm 812 may be trained to learn meaningful, representations or embeddings 814 generally by first translating or encoding input volumes 801 into the representations or embeddings 814, and then minimizing a contrastive loss between representations or embeddings 814. In some embodiments, the representations or embeddings 814 may alone provide an indication of a presence of ARIA (e.g., "1", "2", or "3" in the simplified 3-point scoring mechanism) or an absence of ARIA (e.g., "0" in the simplified 3-point scoring mechanism). Indeed, through the foregoing contrastive learning (e.g., supervised contrastive learning, self-supervised contrastive learning) pre-training of the pre-training classification model 808B, the pre-training classification model 808B may be trained to detect the presence or absence of ARIA, without any use of, or with only limited use of, class labeled or annotated training data, and may further reduce potential model overfitting that may occur due to training with only a limited training data set.

In certain embodiments, subsequent to the pre-training of the pre-training classification model 808B, the representations or embeddings 814 may be then utilized to generate one or more classification scores (e.g., one or more classification scores 810 as discussed above with respect to FIG. 8A) indicative of a severity of ARIA. For example, in some embodiments, the representations or embeddings 814 may be utilized to generate one or more classification scores indicative of mild ARIA (e.g., "0" or "1" in the simplified 3-point scoring mechanism) or severe ARIA (e.g., "2" or "3" in the simplified 3-point scoring mechanism). Specifically, in certain embodiments, subsequent to the pre-training of the pre-training classification model 808B, another fully connected layer (e.g., of size embedding dimensionality *n* x 1 for binary classification or suitable size) and sigmoid layer (e.g., as each included in the classification model 808A discussed above with respect to FIG. 8A) may be added to the pre-training classification model 808B. The added fully connected layer and sigmoid layer (e.g., as each included in the classification model 808A discussed above with respect to FIG. 8A) may be then trained by optimizing the weights of the added fully connected layer and fixing the weights of all the other layers of the pre-training classification model 808B, for example.

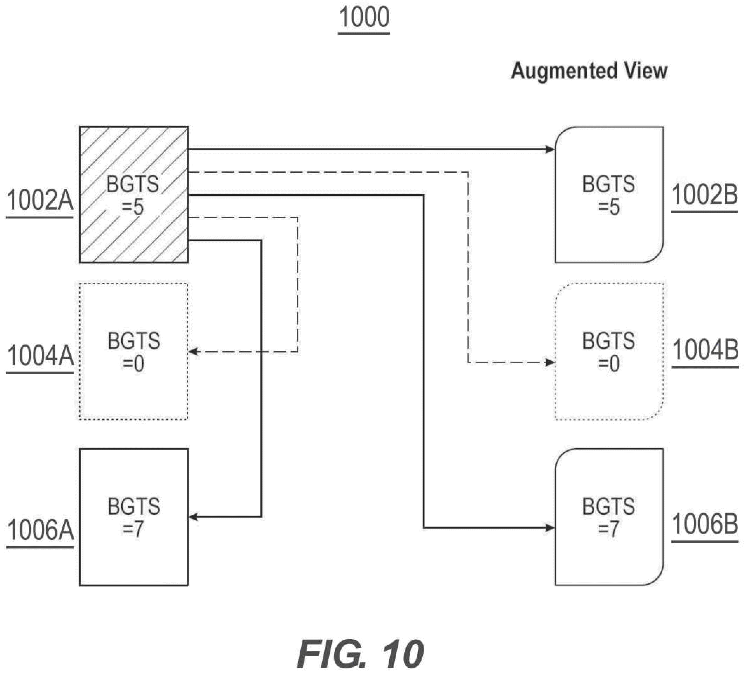

**FIG. 9** illustrates an exemplary process 900 for pre-training a classification model (e.g., pre-training classification model 808B as discussed above with respect to FIG. 8B) using contrastive learning techniques, in accordance with some embodiments. At block 902, one or more computing devices may access a set of brain-scan images associated with one or more patients, in which the set of brain-scan images may include at least a first image of a first ARIA patient's brain, a second image of a second ARIA patient's brain, and a third image of a third patient's brain without ARIA. For example, in certain embodiments, the first ARIA patient's brain may be similar to the second image of the second ARIA patient's brain. In some embodiments, the third image may include an image of AD patient's brain without ARIA, and the third image may be dissimilar to both the first image and the second image, as well as dissimilar to augmentations of the first image and the second image. For example, as to be discussed below with respect to FIG. 10, the set of brain-scan images for pre-training the pre-training classification model 808B may include a set of original images and one or more augmented versions of the original images. In some embodiments, the contrastive learning (e.g., supervised contrastive learning, self-supervised contrastive learning) pre-training of the classification model 808B may be based on training data comprising positive examples of ARIA and negative examples of ARIA. For example, for the contrastive learning (e.g., supervised contrastive learning, self-supervised contrastive learning) of mild (e.g., BGTS score <= "4") vs severe (e.g., BGTS score > "4") ARIA classification, the presence (e.g., BGTS score > "0") or absence of ARIA (e.g., BGTS score = "0") may be used as the auxiliary task.

At block 904, the one or more computing devices may then input the set of brain-scan images into a machine-learning model (e.g., pre-training classification model 808B) to generate a first representation (e.g., representations or embeddings 814) based on the first image and a first augmentation of the first image, a second representation (e.g., representations or embeddings 814) based on the second image and a second augmentation of the second image, and a third representation (e.g., representations or embeddings 814) based on the third image and a third augmentation of the third image. For example, as will be further appreciated with respect to FIG. 10 below, the first representation may include representations or embeddings 814 learned from the first image of the first ARIA patient's brain and one or more augmentations of the first image, the second representation may include representations or embeddings 814 learned from the second image of the second ARIA patient's brain and one or more augmentations of the second image, and the third representation may include representations or embeddings 814 learned from the third image of the AD patient's brain and one or more augmentations the set of brain-scan images for pre-training the pre-training classification model 808B may include a set of original images and one or more augmented versions of the original images. In some embodiments, the augmentations of the first image, the second image, and third image may include, for example, one or more image augmentations, such as a scaling, a rotation, a translation, a cropping, a recoloring, a resizing, a resolution adjustment, a brightness adjustment, a contrast adjustment, an exposure adjustment, or other similar augmentation suitable for at least partially distinguishing the augmented versions of the first image, the second image, and the third image from the original versions of the first image, the second image, and the third image, such that the pre-training classification model 808B learns over time the similarity and/or dissimilarity between the images and their corresponding augmented versions.

At block 906, the one or more computing devices may then determine one or more contrastive losses between the first representation, the second representation, and the third representation by comparing: 1) a similarity between the first representation and the second representation, and 2) a dissimilarity between the third representation and at least one of the first representation or the second representation. For example, in certain embodiments, during training, the contrastive loss function may be utilized to minimize the distance (e.g., maximizing similarity) between the representations of the similar images (e.g., the first image and the second image) while maximizing the distance (e.g., maximizing dissimilarity) between the third image and the first image and the second image that are each dissimilar to the third image. Accordingly, the one or more computing devices may improve the generality of learned representations.

In some embodiments, the contrastive loss function for self-supervised learning (SSL) may be the one shown below: ${L}^{self} = {\sum }_{i ∈ I} {L}_{i}^{self} = - {\sum }_{i ∈ I} log \frac{exp \left({z}_{i}⋅{z}_{j \left(i\right)}/τ\right)}{{\sum }_{a ∈ A \left(i\right)} exp \left({z}_{i}⋅{z}_{a}/τ\right)}$

The batch may include a set of N input image and target class label pairs, which are then augmented to get a different view of the same N input image and target class label pairs, leading to a total of 2*N* input-target pairs in the batch. For self-supervised learning, the positive samples include i, which a selected sample or anchor from the batch, and j(i), which is the augmented or other pair of i. The negative samples is A(i), which are the set of pairs that do not include the anchor and its augmented input-target pairs. The numerator is the dot product of the representation of the positive samples and the denominator is the dot product of the representation of the anchor sample with other samples (and their augmentations) in the negative set.

The above self-supervised loss is extended for the supervised contrastive learning setting and the loss function is shown below: ${L}_{out}^{sup} = {\sum }_{i ∈ I} {L}_{out , i}^{sup} = {\sum }_{i ∈ I} \frac{- 1}{\left|P\left(i\right)\right|} {\sum }_{p ∈ P \left(i\right)} log \frac{exp \left({z}_{i}⋅{z}_{p}/τ\right)}{{\sum }_{a ∈ A \left(i\right)} exp \left({z}_{i}⋅{z}_{a}/τ\right)}$

The set P(i) now includes all positive samples that do not include the anchor input-target pair. The numerator includes contributions from all positive samples and encourages similar representation to all samples from the same class.

Another variation of the supervised contrastive loss is shown below, where the summation term is moved from outside to inside the log function. ${L}_{in}^{sup} = {\sum }_{i ∈ I} {L}_{in , i}^{sup} = {\sum }_{i ∈ I} - log \left\{\frac{1}{\left|P\left(i\right)\right|}{\sum }_{p ∈ P \left(i\right)} \frac{exp \left({z}_{i}⋅{z}_{p}/τ\right)}{{\sum }_{a ∈ A \left(i\right)} exp \left({z}_{i}⋅{z}_{a}/τ\right)}\right\}$

At block 908, the one or more computing devices may then update the machine-learning model (e.g., pre-training classification model 808B) based on the one or more contrastive losses. For example, in some embodiments, updating the machine-learning model based on the one or more contrastive losses may include maximizing the similarity between the first representation and the second representation and maximizing the dissimilarity between the third representation and the at least one of the first representation or the second representation. Specifically, as previously noted in the examples above, one or more of the contrastive loss functions described above may be utilized to iteratively minimize the distance (e.g., maximizing similarity) between the representations of the samples from the same and/or similar class while maximizing the distance (e.g., maximizing dissimilarity) between samples of the dissimilar classes.

**FIG. 10** illustrates exemplary training images 1000 used for contrastive learning. As shown, the training images 1000 include a first original medical image 1002A depicting a positive class of ARIA (where BGTS = "5"), an augmented version of the first original medical image 1002B, a second original medical image 1004A depicting a negative class of ARIA (where BGTS = 0), an augmented version of the second original medical image 1004B, a third original medical image 1006A depicting another example of positive class of ARIA (where BGTS = 7), and an augmented version of the third original medical image 1006B. It should be appreciated that the positive examples and negative examples of ARIA may be defined differently. Taking first original medical image 1002A as the anchor, the solid lines show the pairs for which the output embeddings should be closer and the dotted lines show the pairs for which the output embeddings should be well separated.

In some embodiments, the one or more computing devices may progressively train the network by increasing the complexity of the problem. For example, a training technique that utilizes well separated positive and negative samples during the initial stages (e.g., contrasting cases without ARIA with moderate or severe ARIA cases) and gradually reducing the distance between positive and negative samples (e.g., contrasting cases without ARIA with mild ARIA cases) to provide hard negative mining may be used for a classification model that detects ARIA (yes / no binary outcome).

**FIG. 11** illustrates an exemplary stack of 32 MRI scans 1100 of a patient's brain at 32 different cross sections of the brain. The stack of MRI scans 1100 may be used in block 302 of FIG. 3A or block 702 in FIG. 7. As another example, **FIG. 12** illustrates an exemplary stack of 32 MRI scans 1200 of another patient's brain at 32 different cross sections of the brain. The stack of MRI scans 1200 may be used in block 302 of FIG. 3A or block 702 in FIG. 7.

**FIG. 13** illustrates an example of one or more computing device(s) 1300 that may be utilized to segment, detect, and quantify amyloid-related imaging abnormalities (ARIA) in Alzheimer's disease (AD) patients, in accordance with the presently disclosed embodiments. In certain embodiments, the one or more computing device(s) 1300 may perform one or more steps of one or more methods described or illustrated herein. In certain embodiments, the one or more computing device(s) 1300 provide functionality described or illustrated herein. In certain embodiments, software running on the one or more computing device(s) 1300 performs one or more steps of one or more methods described or illustrated herein or provides functionality described or illustrated herein. Certain embodiments include one or more portions of the one or more computing device(s) 1300.

This disclosure contemplates any suitable number of computing systems to be used as computing device(s) 1300. This disclosure contemplates one or more computing device(s) 1300 taking any suitable physical form. As example and not by way of limitation, one or more computing device(s) 1300 may be an embedded computer system, a system-on-chip (SOC), a single-board computer system (SBC) (e.g., a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, a tablet computer system, an augmented/virtual reality device, or a combination of two or more of these. Where appropriate, the one or more computing device(s) 1300 may be unitary or distributed; span multiple locations; span multiple machines; span multiple data centers; or reside in a cloud, which may include one or more cloud components in one or more networks.

Where appropriate, the one or more computing device(s) 1300 may perform without substantial spatial or temporal limitation one or more steps of one or more methods described or illustrated herein. As an example, and not by way of limitation, the one or more computing device(s) 1300 may perform in real time or in batch mode one or more steps of one or more methods described or illustrated herein. The one or more computing device(s) 1300 may perform at different times or at different locations one or more steps of one or more methods described or illustrated herein, where appropriate.

In certain embodiments, the one or more computing device(s) 1300 includes a processor 1302, memory 1304, database 1306, an input/output (I/O) interface 1308, a communication interface 1310, and a bus 1312. Although this disclosure describes and illustrates a particular computer system having a particular number of particular components in a particular arrangement, this disclosure contemplates any suitable computer system having any suitable number of any suitable components in any suitable arrangement. In certain embodiments, processor 1302 includes hardware for executing instructions, such as those making up a computer program. As an example, and not by way of limitation, to execute instructions, processor 1302 may retrieve (or fetch) the instructions from an internal register, an internal cache, memory 1304, or database 1306; decode and execute them; and then write one or more results to an internal register, an internal cache, memory 1304, or database 1306. In certain embodiments, processor 1302 may include one or more internal caches for data, instructions, or addresses. This disclosure contemplates processor 1302 including any suitable number of any suitable internal caches, where appropriate. As an example, and not by way of limitation, processor 1302 may include one or more instruction caches, one or more data caches, and one or more translation lookaside buffers (TLBs). Instructions in the instruction caches may be copies of instructions in memory 1304 or database 1306, and the instruction caches may speed up retrieval of those instructions by processor 1302.

Data in the data caches may be copies of data in memory 1304 or database 1306 for instructions executing at processor 1302 to operate on; the results of previous instructions executed at processor 1302 for access by subsequent instructions executing at processor 1302 or for writing to memory 1304 or database 1306; or other suitable data. The data caches may speed up read or write operations by processor 1302. The TLBs may speed up virtual-address translation for processor 1302. In certain embodiments, processor 1302 may include one or more internal registers for data, instructions, or addresses. This disclosure contemplates processor 1302 including any suitable number of any suitable internal registers, where appropriate. Where appropriate, processor 1302 may include one or more arithmetic logic units (ALUs); be a multi-core processor; or include one or more processors 1302. Although this disclosure describes and illustrates a particular processor, this disclosure contemplates any suitable processor.

In certain embodiments, memory 1304 includes main memory for storing instructions for processor 1302 to execute or data for processor 1302 to operate on. As an example, and not by way of limitation, the one or more computing device(s) 1300 may load instructions from database 1306 or another source (such as, for example, another one or more computing device(s) 1300) to memory 1304. Processor 1302 may then load the instructions from memory 1304 to an internal register or internal cache. To execute the instructions, processor 1302 may retrieve the instructions from the internal register or internal cache and decode them. During or after execution of the instructions, processor 1302 may write one or more results (which may be intermediate or final results) to the internal register or internal cache. Processor 1302 may then write one or more of those results to memory 1304.

In certain embodiments, processor 1302 executes only instructions in one or more internal registers or internal caches or in memory 1304 (as opposed to database 1306 or elsewhere) and operates only on data in one or more internal registers or internal caches or in memory 1304 (as opposed to database 1306 or elsewhere). One or more memory buses (which may each include an address bus and a data bus) may couple processor 1302 to memory 1304. Bus 1312 may include one or more memory buses, as described below. In certain embodiments, one or more memory management units (MMUs) reside between processor 1302 and memory 1304 and facilitate accesses to memory 1304 requested by processor 1302. In certain embodiments, memory 1304 includes random access memory (RAM). This RAM may be volatile memory, where appropriate. Where appropriate, this RAM may be dynamic RAM (DRAM) or static RAM (SRAM). Moreover, where appropriate, this RAM may be singleported or multi-ported RAM. This disclosure contemplates any suitable RAM. Memory 1304 may include one or more memory devices 1304, where appropriate. Although this disclosure describes and illustrates particular memory, this disclosure contemplates any suitable memory.

In certain embodiments, database 1306 includes mass storage for data or instructions. As an example, and not by way of limitation, database 1306 may include a hard disk drive (HDD), a floppy disk drive, flash memory, an optical disc, a magneto-optical disc, magnetic tape, or a Universal Serial Bus (USB) drive or a combination of two or more of these. Database 1306 may include removable or non-removable (or fixed) media, where appropriate. Database 1306 may be internal or external to the one or more computing device(s) 1300, where appropriate. In certain embodiments, database 1306 is non-volatile, solid-state memory. In certain embodiments, database 1306 includes read-only memory (ROM). Where appropriate, this ROM may be mask-programmed ROM, programmable ROM (PROM), erasable PROM (EPROM), electrically erasable PROM (EEPROM), electrically alterable ROM (EAROM), or flash memory or a combination of two or more of these. This disclosure contemplates mass database 1306 taking any suitable physical form. Database 1306 may include one or more storage control units facilitating communication between processor 1302 and database 1306, where appropriate. Where appropriate, database 1306 may include one or more databases 1306. Although this disclosure describes and illustrates particular storage, this disclosure contemplates any suitable storage.

In certain embodiments, I/O interface 1308 includes hardware, software, or both, providing one or more interfaces for communication between the one or more computing device(s) 1300 and one or more I/O devices. The one or more computing device(s) 1300 may include one or more of these I/O devices, where appropriate. One or more of these I/O devices may enable communication between a person and the one or more computing device(s) 1300. As an example, and not by way of limitation, an I/O device may include a keyboard, keypad, microphone, monitor, mouse, printer, scanner, speaker, still camera, stylus, tablet, touch screen, trackball, video camera, another suitable I/O device or a combination of two or more of these. An I/O device may include one or more sensors. This disclosure contemplates any suitable I/O devices and any suitable I/O interfaces 1308 for them. Where appropriate, I/O interface 1308 may include one or more device or software drivers enabling processor 1302 to drive one or more of these I/O devices. I/O interface 1308 may include one or more I/O interfaces 1308, where appropriate. Although this disclosure describes and illustrates a particular I/O interface, this disclosure contemplates any suitable I/O interface.

In certain embodiments, communication interface 1310 includes hardware, software, or both providing one or more interfaces for communication (such as, for example, packet-based communication) between the one or more computing device(s) 1300 and one or more other computing device(s) 1300 or one or more networks. As an example, and not by way of limitation, communication interface 1310 may include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 1310 for it.

As an example, and not by way of limitation, the one or more computing device(s) 1300 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, the one or more computing device(s) 1300 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination of two or more of these. The one or more computing device(s) 1300 may include any suitable communication interface 1310 for any of these networks, where appropriate. Communication interface 1310 may include one or more communication interfaces 1310, where appropriate. Although this disclosure describes and illustrates a particular communication interface, this disclosure contemplates any suitable communication interface.

In certain embodiments, bus 1312 includes hardware, software, or both coupling components of the one or more computing device(s) 1300 to each other. As an example, and not by way of limitation, bus 1312 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination of two or more of these. Bus 1312 may include one or more buses 1312, where appropriate. Although this disclosure describes and illustrates a particular bus, this disclosure contemplates any suitable bus or interconnect.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

**FIG. 14** illustrates a diagram 1400 of an example artificial intelligence (AI) architecture 1402 (which may be included as part of the one or more computing device(s) 1300 as discussed above with respect to FIG. 6) that may be utilized to segment, detect, and quantify amyloid-related imaging abnormalities (ARIA) in Alzheimer's disease (AD) patients, in accordance with the presently disclosed embodiments. In certain embodiments, the AI architecture 1402 may be implemented utilizing, for example, one or more processing devices that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), and/or other processing device(s) that may be suitable for processing various medical data and making one or more decisions based thereon), software (e.g., instructions running/executing on one or more processing devices), firmware (e.g., microcode), or some combination thereof.

In certain embodiments, as depicted by FIG. 14, the AI architecture 1402 may include machine learning (ML) algorithms and functions 1404, natural language processing (NLP) algorithms and functions 1406, expert systems 1408, computer-based vision algorithms and functions 1410, speech recognition algorithms and functions 1412, planning algorithms and functions 1414, and robotics algorithms and functions 1416. In certain embodiments, the ML algorithms and functions 1404 may include any statistics-based algorithms that may be suitable for finding patterns across large amounts of data (e.g., "Big Data" such as genomics data, proteomics data, metabolomics data, metagenomics data, transcriptomics data, medication data, medical diagnostics data, medical procedures data, medical diagnoses data, medical symptoms data, demographics data, patient lifestyle data, physical activity data, family history data, socioeconomics data, geographic environment data, and so forth). For example, in certain embodiments, the ML algorithms and functions 1404 may include deep learning algorithms 1418, supervised learning algorithms 1420, and unsupervised learning algorithms 1422.

In certain embodiments, the deep learning algorithms 1418 may include any artificial neural networks (ANNs) that may be utilized to learn deep levels of representations and abstractions from large amounts of data. For example, the deep learning algorithms 1418 may include ANNs, such as a perceptron, a multilayer perceptron (MLP), an autoencoder (AE), a convolution neural network (CNN), a recurrent neural network (RNN), long short term memory (LSTM), a grated recurrent unit (GRU), a restricted Boltzmann Machine (RBM), a deep belief network (DBN), a bidirectional recurrent deep neural network (BRDNN), a generative adversarial network (GAN), and deep Q-networks, a neural autoregressive distribution estimation (NADE), an adversarial network (AN), attentional models (AM), a spiking neural network (SNN), deep reinforcement learning, and so forth.

In certain embodiments, the supervised learning algorithms 1420 may include any algorithms that may be utilized to apply, for example, what has been learned in the past to new data using labeled examples for predicting future events. For example, starting from the analysis of a known training data set, the supervised learning algorithms 1420 may produce an inferred function to make predictions about the output values. The supervised learning algorithms 1420 may also compare its output with the correct and intended output and find errors in order to modify the supervised learning algorithms 1420 accordingly. On the other hand, the unsupervised learning algorithms 1422 may include any algorithms that may applied, for example, when the data used to train the unsupervised learning algorithms 1422 are neither classified nor labeled. For example, the unsupervised learning algorithms 1422 may study and analyze how systems may infer a function to describe a hidden structure from unlabeled data.

In certain embodiments, the NLP algorithms and functions 1406 may include any algorithms or functions that may be suitable for automatically manipulating natural language, such as speech and/or text. For example, in some embodiments, the NLP algorithms and functions 1406 may include content extraction algorithms or functions 1424, classification algorithms or functions 1426, machine translation algorithms or functions 1428, question answering (QA) algorithms or functions 1430, and text generation algorithms or functions 1432. In certain embodiments, the content extraction algorithms or functions 1424 may include a means for extracting text or images from electronic documents (e.g., webpages, text editor documents, and so forth) to be utilized, for example, in other applications.

In certain embodiments, the classification algorithms or functions 1426 may include any algorithms that may utilize a supervised learning model (e.g., logistic regression, naive Bayes, stochastic gradient descent (SGD), k-nearest neighbors, decision trees, random forests, support vector machine (SVM), and so forth) to learn from the data input to the supervised learning model and to make new observations or classifications based thereon. The machine translation algorithms or functions 1428 may include any algorithms or functions that may be suitable for automatically converting source text in one language, for example, into text in another language. The QA algorithms or functions 1430 may include any algorithms or functions that may be suitable for automatically answering questions posed by humans in, for example, a natural language, such as that performed by voice-controlled personal assistant devices. The text generation algorithms or functions 1432 may include any algorithms or functions that may be suitable for automatically generating natural language texts.

In certain embodiments, the expert systems 1408 may include any algorithms or functions that may be suitable for simulating the judgment and behavior of a human or an organization that has expert knowledge and experience in a particular field (e.g., stock trading, medicine, sports statistics, and so forth). The computer-based vision algorithms and functions 1410 may include any algorithms or functions that may be suitable for automatically extracting information from images (e.g., photo images, video images). For example, the computer-based vision algorithms and functions 1410 may include image recognition algorithms 1434 and machine vision algorithms 1436. The image recognition algorithms 1434 may include any algorithms that may be suitable for automatically identifying and/or classifying objects, places, people, and so forth that may be included in, for example, one or more image frames or other displayed data. The machine vision algorithms 1436 may include any algorithms that may be suitable for allowing computers to "see", or, for example, to rely on image sensors cameras with specialized optics to acquire images for processing, analyzing, and/or measuring various data characteristics for decision making purposes.

In certain embodiments, the speech recognition algorithms and functions 1412 may include any algorithms or functions that may be suitable for recognizing and translating spoken language into text, such as through automatic speech recognition (ASR), computer speech recognition, speech-to-text (STT) 1438, or text-to-speech (TTS) 1440 in order for the computing to communicate via speech with one or more users, for example. In certain embodiments, the planning algorithms and functions 1414 may include any algorithms or functions that may be suitable for generating a sequence of actions, in which each action may include its own set of preconditions to be satisfied before performing the action. Examples of AI planning may include classical planning, reduction to other problems, temporal planning, probabilistic planning, preference-based planning, conditional planning, and so forth. Lastly, the robotics algorithms and functions 1416 may include any algorithms, functions, or systems that may enable one or more devices to replicate human behavior through, for example, motions, gestures, performance tasks, decision-making, emotions, and so forth.

Herein, "or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

Herein, "automatically" and its derivatives means "without human intervention," unless expressly indicated otherwise or indicated otherwise by context.

The embodiments disclosed herein are only examples, and the scope of this disclosure is not limited to them. Embodiments according to this disclosure are in particular disclosed in the attached claims directed to a method, a storage medium, a system and a computer program product, wherein any feature mentioned in one claim category, e.g., method, may be claimed in another claim category, e.g., system, as well. The dependencies or references back in the attached claims are chosen for formal reasons only. However, any subject matter resulting from a deliberate reference back to any previous claims (in particular multiple dependencies) may be claimed as well, so that any combination of claims and the features thereof are disclosed and may be claimed regardless of the dependencies chosen in the attached claims. The subjectmatter which may be claimed comprises not only the combinations of features as set out in the attached claims but also any other combination of features in the claims, wherein each feature mentioned in the claims may be combined with any other feature or combination of other features in the claims. Furthermore, any of the embodiments and features described or depicted herein may be claimed in a separate claim and/or in any combination with any embodiment or feature described or depicted herein or with any of the features of the attached claims.

The scope of this disclosure encompasses all changes, substitutions, variations, alterations, and modifications to the example embodiments described or illustrated herein that a person having ordinary skill in the art would comprehend. The scope of this disclosure is not limited to the example embodiments described or illustrated herein. Moreover, although this disclosure describes and illustrates respective embodiments herein as including particular components, elements, feature, functions, operations, or steps, any of these embodiments may include any combination or permutation of any of the components, elements, features, functions, operations, or steps described or illustrated anywhere herein that a person having ordinary skill in the art would comprehend. Furthermore, reference in the appended claims to an apparatus or system or a component of an apparatus or system being adapted to, arranged to, capable of, configured to, enabled to, operable to, or operative to perform a particular function encompasses that apparatus, system, component, whether or not it or that particular function is activated, turned on, or unlocked, as long as that apparatus, system, or component is so adapted, arranged, capable, configured, enabled, operable, or operative. Additionally, although this disclosure describes or illustrates certain embodiments as providing particular advantages, certain embodiments may provide none, some, or all of these advantages.

### EXAMPLE EMBODIMENTS

Embodiments disclosed herein may include:
1. A method for quantifying amyloid related imaging abnormalities (ARIA) in a brain of a patient, comprising, by one or more computing devices: accessing a set of one or more brain-scan images associated with the patient; inputting the set of one or more brain-scan images into one or more machine-learning models trained to generate a segmentation map based on the set of one or more brain-scan images, the segmentation map including a plurality of pixel-wise class labels corresponding to a plurality of pixels in the segmentation map, wherein at least one of the plurality of pixel-wise class labels comprises an indication of ARIA in the brain of the patient; and outputting a quantification of ARIA in the brain of the patient based at least in part on the segmentation map.
2. The method of embodiment 1, wherein the ARIA is associated with microhemorrhages and hemosiderin deposits (ARIA-H) in the brain of the patient.
3. The method of embodiment 1, wherein the ARIA is associated parenchymal edema or sulcal effusion (ARIA-E) in the brain of the patient.
4. The method of any one of embodiments 1-3, wherein the patient is an Alzheimer's disease (AD) patient having been treated with an anti-amyloid-beta (anti-Aβ) antibody.
5. The method of embodiment 4, further comprising: in response to outputting the quantification of ARIA in the brain of the patient, determining a dosage adjustment of the anti-Aβ antibody.
6. The method of embodiment 4 or 5, further comprising: in response to outputting the quantification of ARIA in the brain of the patient, terminating or temporarily suspending use of the anti-Aβ antibody in the patient.
7. The method of any one of embodiments 4-6, wherein the anti-Aβ antibody is selected from the group consisting of bapineuzumab, solanezumab, aducanumab, gantenerumab, crenezumab, donanemab, and lecanemab.
8. The method of any one of embodiments 1-7, further comprising: in response to outputting the quantification of ARIA in the brain of the patient, determining one or more anti-ARIA treatments for the patient.
9. The method of embodiment 8, further comprising: administering the one or more anti-ARIA treatments to the patient.
10. The method of any one of embodiments 8-9, wherein the one or more anti-ARIA treatments comprise one or more anti-ARIA antibodies.
11. The method of any one of embodiments 1-10, wherein the set of one or more brain-scan images comprises one or more magnetic resonance imaging (MRI) images, one or more positron emission tomography (PET) images, one or more single-photon emission computed tomography (SPECT) images, one or more amyloid PET images, or any combination thereof.
12. The method of any one of embodiments 1-10, wherein the set of one or more brain-scan images comprises one or more fluid-attenuated inversion recovery (FLAIR) images, one or more T2*-weighted imaging (T2*WI) images, one or more T1-weighted imaging (T1WI) images, or any combination thereof.
13. The method of any one of embodiments 1-12, wherein the one or more machine-learning models comprises: an encoder trained to generate a plurality of down-sampled feature maps based on the set of one or more brain-scan images; and a decoder trained to: generate a plurality of up-sampled feature maps based on the plurality of down-sampled feature maps; and generate the segmentation map based on the plurality of up-sampled feature maps.
14. The method of embodiment 13, wherein the encoder comprises a neural network.
15. The method of embodiment 13, wherein the encoder comprises a harmonic dense neural network (HarDNet) encoder.
16. The method of embodiment 13, wherein the decoder comprises a neural network.
17. The method of Embodiment 13, wherein the decoder comprises a U-Net decoder.
18. The method of any one of embodiments 1-17, wherein the one or more machine-learning models is trained using image augmentations.
19. The method of any one of embodiments 1-18, wherein the at least one of the plurality of pixel-wise class labels comprises an indication of one or more ARIA lesions.
20. The method of embodiment 19, wherein the one or more machine-learning models comprises a segmentation model comprising an encoder trained to generate a plurality of down-sampled feature maps based on the set of one or more brain-scan images, the method further comprising: detecting ARIA in the brain of the patient by generating, utilizing a classification model associated with the segmentation model, a classification score based at least in part on the plurality of down-sampled feature maps.
21. A method for pre-training one or more classification models for detecting amyloid related imaging abnormalities (ARIA) in brains of patients, comprising, by one or more computing devices: accessing a set of brain-scan images associated with one or more patients, wherein the set of brain-scan images comprises at least a first image of a first ARIA patient's brain, a second image of a second ARIA patient's brain, and a third image of a third patient's brain without ARIA; inputting the set of brain-scan images into a machine-learning model to generate a first representation based on the first image and a first augmentation of the first image, a second representation based on the second image and a second augmentation of the second image, and a third representation based on the third image and a third augmentation of the third image; determining one or more contrastive losses between the first representation, the second representation, and the third representation by comparing: 1) a similarity between the first representation and the second representation, and 2) a dissimilarity between the third representation and at least one of the first representation or the second representation; and updating the machine-learning model based on the one or more contrastive losses.
22. The method of embodiment 21, wherein the third image comprises an image of an Alzheimer's disease (AD) patient's brain without ARIA.
23. The method of embodiment 21, wherein the first image is similar to the second image, and wherein the third image is dissimilar to first image and the second image.
24. The method of embodiment 23, wherein: the first image comprises a positive class of ARIA; the first augmentation of the first image comprises an augmented version of the first image; the second image comprises a positive class of ARIA; the second augmentation of the second image comprises an augmented version of the second image; the third image comprises a negative class of ARIA; and the third augmentation of the third image comprises an augmented version of the third image.
25. The method of any one of embodiments 21-24, wherein the machine-learning model comprises a supervised contrastive-learning model.
26. The method of any one of embodiments 21-24, wherein the machine-learning model comprises a self-supervised contrastive-learning model.
27. The method of any one of embodiments 21-26, wherein updating the machine-learning model based on the one or more contrastive losses comprises maximizing the similarity between the first representation and the second representation.
28. The method of any one of embodiments 21-26, wherein updating the machine-learning model based on the one or more contrastive losses comprises minimizing a distance between the first representation and the second representation.
29. The method of any one of embodiments 21-26, wherein updating the machine-learning model based on the one or more contrastive losses comprises minimizing a similarity between the third representation and the at least one of the first representation or the second representation.
30. The method of any one of embodiments 21-26, wherein updating the machine-learning model based on the one or more contrastive losses comprises maximizing the dissimilarity between the third representation and the at least one of the first representation or the second representation.
31. The method of any one of embodiments 21-30, wherein determining the one or more contrastive losses further comprises determining a contrastive loss between one or more of: the first representation and the first augmentation of the first image; the second representation and the second augmentation of the second image; and the third representation and the third augmentation of the third image.
32. The method of embodiment 31, wherein updating the machine-learning model further comprises: maximizing a similarity between the first representation and the first augmentation of the first image; maximizing a similarity between the second representation and the second augmentation of the second image; and maximizing a similarity between the third representation and the third augmentation of the third image.
33. The method of any one of embodiments 21-30, further comprising training the updated machine-learning model to generate one or more classification scores indicative of ARIA.
34. The method of embodiment 33, wherein the one or more classification scores comprise a binary value indicative of an absence of ARIA or a presence of ARIA.
35. The method of embodiment 33, wherein the one or more classification scores comprise a numerical value indicative of a severity of ARIA.
36. The method of embodiment 33, wherein the one or more classification scores comprise one of a plurality of classification scores, and wherein the plurality of classification scores comprises: a first classification score indicative of mild ARIA; a second classification score indicative of moderate ARIA; and a third classification score indicative of severe ARIA.
37. The method of embodiment 33, wherein the one or more classification scores comprise a Barkhof Grand Total Score (BGTS) score.
38. The method of any one of embodiments 21-37, further comprising: accessing a second set of brain-scan images associated with another patient; inputting the second set of brain-scan images into the updated machine-learning model further trained to generate a classification score based on the second set of brain-scan images; and detecting a presence of ARIA or an absence of ARIA in a brain of the other patient based on the classification score.
39. The method of any one of embodiments 21-38, further comprising: accessing a second set of brain-scan images associated with another patient; inputting the second set of brain-scan images into the updated machine-learning model further trained to generate a classification score based on the second set of brain-scan images; and determining a severity of ARIA in a brain of the other patient based on the classification score.
40. The method of any one of embodiments 21-39, wherein the machine-learning model comprises a classification model.
41. The method of embodiment 40, wherein the classification model comprises an encoder.
42. The method of embodiment 41, wherein the encoder comprises a harmonic dense neural network (HarDNet).
43. The method of any one of embodiments 40-42, wherein updating the machine-learning model further comprises: pre-training the classification model on a self-supervised learning (SSL) task based on the set of brain-scan images, and after pre-training the classification model on the SSL task, training the classification model on a classification task or a regression task based on the SSL task.
44. A system including one or more computing devices, comprising: one or more non-transitory computer-readable storage media including instructions; and one or more processors coupled to the one or more storage media, the one or more processors configured to execute the instructions to perform the method of any one of embodiments 1-43.
45. A non-transitory computer-readable medium comprising instructions that, when executed by one or more processors of one or more computing devices, cause the one or more processors to effectuate the method of any one of embodiments 1-43.

### FURTHER EXAMPLE EMBODIMENTS

Further embodiments disclosed herein may include:
1. A method for detecting amyloid related imaging abnormalities (ARIA) in a brain of a patient, comprising, by one or more computing devices: accessing a set of one or more brain-scan images associated with the patient; inputting the set of one or more brain-scan images into one or more machine-learning models trained to: generate a segmentation map based on the set of one or more brain-scan images, the segmentation map including a plurality of pixel-wise class labels corresponding to a plurality of pixels in the segmentation map; and generate a classification score based on the segmentation map; and detecting ARIA in the brain of the patient based on the classification score.
2. The method of embodiment 1, wherein the ARIA is associated with microhemorrhages and hemosiderin deposits (ARIA-H) in the brain of the patient.
3. The method of any one of embodiments 1-2, wherein the ARIA is associated parenchymal edema or sulcal effusion (ARIA-E) in the brain of the patient.
4. The method of any one of embodiments 1-3, wherein the one or more machine-learning models comprises a segmentation model and a classification model.
5. The method of embodiment 4, wherein the segmentation model comprises an encoder trained to generate a plurality of down-sampled feature maps based on the set of one or more brain-scan images, and wherein the classification model comprises a decoder trained to receive the plurality of down-sampled feature maps from the encoder.
6. The method of any one of embodiments 4-5, wherein the segmentation model further comprises a bidirectional feature propagation network.
7. The method of embodiment 6, wherein the bidirectional feature propagation network comprises a top-down feature pyramid network (FPN) and a bottom-up FPN.
8. The method of embodiment 7, wherein the classification model comprises a decoder trained to receive input data from a plurality of layers of the bottom-up FPN.
9. The method of any of embodiments 4-8, wherein the classification model is trained by:
   training the segmentation model; and after training the segmentation model, training the classification model based on the trained segmentation model.
10. The method of any of embodiments 4-9, wherein the classification model comprises an attention mechanism.
11. The method of embodiment 10, wherein the attention mechanism is configured to pay attention to one or more of the plurality of pixel-wise class labels included in the segmentation map.
12. The method of any one of embodiments 10-11, wherein the plurality of pixel-wise class labels comprises a first plurality of pixel-wise class labels, and wherein the attention mechanism is based on a second plurality of pixel-wise class labels included in the segmentation map, the second plurality of pixel-wise class labels being indicative of dilated gray matter.
13. The method of any one of embodiments 10-12, wherein the attention mechanism is based on a plurality of subtraction masks.
14. The method of any of embodiments 1-13, wherein the patient is an Alzheimer's disease (AD) patient having been treated with an anti-amyloid-beta (anti-Aβ) antibody.
15. The method of embodiment 14, further comprising: in response to detecting the ARIA in the brain of the patient, determining a dosage adjustment of the anti-Aβ antibody.
16. The method of any of embodiments 14-15, further comprising: in response to detecting the ARIA in the brain of the patient, terminating or temporarily suspending use of the anti-Aβ antibody in the patient.
17. The method of any one of embodiments 14-16, wherein the anti-Aβ antibody is selected from the group consisting of bapineuzumab, solanezumab, aducanumab, gantenerumab, crenezumab, donanemab, and lecanemab.
18. The method of any of embodiments 14-17, further comprising: in response to detecting the ARIA in the brain of the patient, determining one or more anti-ARIA treatments for the patient.
19. The method of embodiment 18, further comprising: administering the one or more anti-ARIA treatments to the patient.
20. The method of any of embodiments 18-19, wherein the one or more anti-ARIA treatments comprise one or more anti-ARIA antibodies.
21. The method of any one of embodiments 1-20, wherein the set of one or more brain-scan images comprises one or more magnetic resonance imaging (MRI) images, one or more positron emission tomography (PET) images, one or more single-photon emission computed tomography (SPECT) images, one or more amyloid PET images, or any combination thereof.
22. The method of any one of embodiments 1-21, wherein the set of one or more brain-scan images comprises one or more fluid-attenuated inversion recovery (FLAIR) images, one or more T2*-weighted imaging (T2*WI) images, one or more T1-weighted imaging (T1WI) images, or any combination thereof.
23. The method of any one of embodiments 1-22, wherein the set of one or more brain-scan images comprises a plurality of volumes corresponding to one or more cross-sectional volumes of the brain of the patient.
24. The method of any one of embodiments 1-23, wherein the classification score comprises a binary value indicative of an absence of ARIA or a presence of ARIA.
25. The method of any one of embodiments 1-24, wherein the classification score comprises a numerical value indicative of a severity of ARIA.
26. The method of any one of embodiments 1-25, wherein the classification score comprises one of a plurality of classification scores, and wherein the plurality of classification scores comprises: a first classification score indicative of mild ARIA; a second classification score indicative of moderate ARIA; and a third classification score indicative of severe ARIA.
27. The method of any one of embodiments 1-26, wherein the classification score comprises a Barkhof Grand Total Score (BGTS) score.
28. The method of any of Embodiments 1-27, wherein at least one of the plurality of pixel-wise class labels comprises an indication of one or more ARIA lesions, and wherein the one or more machine-learning models is further trained to generate the classification score based on the at least one of the plurality of pixel-wise class labels.
29. A method for training a plurality of machine-learning models for detecting amyloid related imaging abnormalities (ARIA) in brains of patients, comprising, by one or more computing devices: accessing a set of brain-scan images associated with one or more patients; training a first machine-learning model of the plurality of machine-learning models, the first machine-learning model being trained to segment one or more ARIA lesions based on the set of brain-scan images; obtaining a first set of weights associated with the trained first machine-learning model; initializing a second set of weights to correspond to the first set of weights, the second set of weights associated with a second machine-learning model of the plurality of machine-learning models; and training the second machine-learning model to generate a classification score based at least in part on the second set of weights, the classification score corresponding to a detection of ARIA or a severity of ARIA in brains of the one or more patients.
30. The method of embodiment 29, wherein the ARIA is associated with microhemorrhages and hemosiderin deposits (ARIA-H) in brains of the one or more patients.
31. The method of any one of embodiments 29-30, wherein the ARIA is associated parenchymal edema or sulcal effusion (ARIA-E) in brains of the one or more patients.
32. The method of any one of embodiments 29-31, wherein the first machine-learning model comprises a segmentation model.
33. The method of embodiment 32, wherein the segmentation model comprises an encoder and a decoder.
34. The method of embodiment 33, wherein the encoder comprises a harmonic dense neural network (HarDNet) encoder.
35. The method of any one of embodiments 33-34, wherein the decoder comprises a U-Net decoder.
36. The method of any one of embodiments 29-35, further comprising: prior to training the second machine-learning model to generate the classification score, inputting the segmented one or more ARIA lesions into the second machine-learning model.
37. The method of any one of embodiments 29-36, wherein the second machine-learning model comprises a classification model.
38. The method of embodiment 37, wherein the classification model comprises an attention mechanism configured to pay attention to one or more features of the set of brain-scan images identified based on the segmented one or more ARIA lesions.
39. The method of any of embodiments 37-38, wherein the classification model comprises one or more convolutional neural networks (CNNs).
40. The method of any one of embodiments 29-39, wherein the classification score comprises a binary value indicative of an absence of ARIA or a presence of ARIA.
41. The method of any one of embodiments 29-40, wherein the classification score comprises a numerical value indicative of a severity of ARIA.
42. The method of any one of embodiments 29-41, wherein the classification score comprises one of a plurality of classification scores, and wherein the plurality of classification scores comprises: a first classification score indicative of mild ARIA; a second classification score indicative of moderate ARIA; and a third classification score indicative of severe ARIA.
43. The method of any one of embodiments 29-42, wherein the classification score comprises a Barkhof Grand Total Score (BGTS) score.
44. The method of any one of embodiments 29-43, wherein training the second machine-learning model comprises optimizing the second set of weights along with the classification score.
45. The method of any one of embodiments 29-44, wherein the first machine-learning model is associated with the second machine-learning model.
46. The method of embodiment 45, wherein training the first machine-learning model and the second machine-learning model comprises training the first machine-learning model and the second machine-learning model in accordance with a transfer learning process.
47. The method of any one of embodiments 29-46, wherein training the second machine-learning model to generate the classification score comprises training the second machine-learning model based on the second set of weights and the segmented one or more ARIA lesions.
48. A system including one or more computing devices, comprising: one or more non-transitory computer-readable storage media including instructions; and one or more processors coupled to the one or more storage media, the one or more processors configured to execute the instructions to perform the method of any one of embodiments 1-47.
49. A non-transitory computer-readable medium comprising instructions that, when executed by one or more processors of one or more computing devices, cause the one or more processors to effectuate the method of any one of embodiments 1-47.

## Claims

1. A method for detecting amyloid related imaging abnormalities, ARIA, in brains of patients, comprising, by one or more computing devices:
accessing a set of brain-scan images associated with a patient;
inputting the set of brain-scan images into a machine-learning model trained to generate a classification score based on the set of brain-scan images; and
determining a presence or absence or severity of ARIA in a brain of the patient based on the classification score;
wherein the machine learning model is a machine learning model that has been pre-trained by:
accessing (902) a set of brain-scan images (801, 1000) associated with one or more patients, wherein the set of brain-scan images comprises at least a first image (1002A) of a first ARIA patient's brain, a second image (1006A) of a second ARIA patient's brain, and a third image (1004A) of a third patient's brain without ARIA;
inputting (904) the set of brain-scan images into a machine-learning model to generate a first representation (814) based on the first image and a first augmentation (1002B) of the first image, a second representation (814) based on the second image and a second augmentation (1006B) of the second image, and a third representation (814) based on the third image and a third augmentation (1004B) of the third image;
determining (906) one or more contrastive losses between the first representation, the second representation, and the third representation by comparing: 1) a similarity between the first representation and the second representation, and 2) a dissimilarity between the third representation and at least one of the first representation or the second representation; and
updating (908) the machine-learning model based on the one or more contrastive losses.

2. The method of Claim 1, wherein the third image comprises an image of an Alzheimer's disease patient's brain without ARIA.

3. The method of Claim 1, wherein the first image is similar to the second image, and wherein the third image is dissimilar to first image and the second image.

4. The method of Claim 3, wherein:
the first image comprises a positive class of ARIA;
the first augmentation of the first image comprises an augmented version of the first image;
the second image comprises a positive class of ARIA;
the second augmentation of the second image comprises an augmented version of the second image;
the third image comprises a negative class of ARIA; and
the third augmentation of the third image comprises an augmented version of the third image.

5. The method of any of Claims 1 to 4, wherein the machine-learning model comprises a supervised contrastive-learning model, or wherein the machine-learning model comprises a self-supervised contrastive-learning model.

6. The method of any of Claims 1 to 5, wherein updating the machine-learning model based on the one or more contrastive losses comprises maximizing the similarity between the first representation and the second representation.

7. The method of any of Claims 1 to 6, wherein updating the machine-learning model based on the one or more contrastive losses comprises: (i) minimizing a distance between the first representation and the second representation; (ii) minimizing a similarity between the third representation and the at least one of the first representation or the second representation; or (iii) maximizing the dissimilarity between the third representation and the at least one of the first representation or the second representation.

8. The method of any of Claims 1 to 7, wherein determining the one or more contrastive losses further comprises determining a contrastive loss between one or more of:
the first representation and the first augmentation of the first image;
the second representation and the second augmentation of the second image; and
the third representation and the third augmentation of the third image.

9. The method of Claim 8, wherein updating the machine-learning model further comprises:
maximizing a similarity between the first representation and the first augmentation of the first image;
maximizing a similarity between the second representation and the second augmentation of the second image; and
maximizing a similarity between the third representation and the third augmentation of the third image.

10. The method of any of Claims 1 to 9, wherein the pre-trained machine learning model has been further trained to generate one or more classification scores indicative of ARIA.

11. The method of Claim 10, wherein the one or more classification scores comprise:
(i) a binary value indicative of an absence of ARIA or a presence of ARIA;
(ii) a numerical value indicative of a severity of ARIA;
(iii) one of a plurality of classification scores, wherein the plurality of classification scores comprises:
a first classification score indicative of mild ARIA;
a second classification score indicative of moderate ARIA; and
a third classification score indicative of severe ARIA; or
(iv) a Barkhof Grand Total Score (BGTS) score.

12. The method of any of Claims 1 to 11, wherein the machine-learning model comprises a classification model, optionally wherein the classification model comprises an encoder or a harmonic dense neural network, HarDNet.

13. The method of Claims 12, wherein the machine-learning model by at least:
pre-training the classification model on a self-supervised learning, SSL, task based on the set of brain-scan images, and
after pre-training the classification model on the SSL task, training the classification model on a classification task or a regression task based on the SSL task.

14. A system including one or more computing devices, comprising:
one or more non-transitory computer-readable storage media including instructions; and
one or more processors coupled to the one or more storage media, the one or more processors configured to execute the instructions to implement the method of any preceding claim.

15. A non-transitory computer-readable medium comprising instructions that, when executed by one or more processors of one or more computing devices, cause the one or more processors to implement the method of any of claims 1 to 14.
